(19) Europäisches Patentamt
European Patent Office
Office européen des brevets

(11) **EP 2 621 516 B1**

(12) **EUROPEAN PATENT SPECIFICATION**

(45) Date of publication and mention
of the grant of the patent:
**22.06.2016 Bulletin 2016/25**

(21) Application number: **11829825.6**

(22) Date of filing: **28.09.2011**

(51) Int Cl.:
**A61K 38/21** (2006.01)    **A61P 35/00** (2006.01)

(86) International application number:
**PCT/US2011/053681**

(87) International publication number:
**WO 2012/044684 (05.04.2012 Gazette 2012/14)**

(54) **INTERFERON-BETA FOR USE AS MONOTHERAPY OR IN COMBINATION WITH OTHER CANCER THERAPIES**

INTERFERON-BETA ZUR VERWENDUNG ALS MONOTHERAPIE ODER IN KOMBINATION MIT ANDEREN KREBSTHERAPIEN

INTERFÉRON-BÊTA UTILISÉ EN TANT QUE MONOTHÉRAPIE OU EN COMBINAISON AVEC D'AUTRES CANCÉROTHÉRAPIES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2010 US 389009 P**

(43) Date of publication of application:
**07.08.2013 Bulletin 2013/32**

(73) Proprietor: **Biogen MA Inc.**
**Cambridge, MA 02142 (US)**

(72) Inventors:
• **BAKER, Darren, P.**
**Hingham, MA 02043 (US)**
• **JOSEPH, Ingrid, B.J.K.**
**San Diego, CA 92128 (US)**
• **WANG, Xinzhong**
**Wayland, MA 01778 (US)**

(74) Representative: **Miller, David James et al**
**Mathys & Squire LLP**
**The Shard**
**32 London Bridge Street**
**London SE1 9SG (GB)**

(56) References cited:
**WO-A2-00/23114          WO-A2-2004/020468**
**WO-A2-2009/036082     US-A1- 2008 003 202**

• **BAKER D P ET AL: "N-terminally PEGylated human interferon -beta-1a with improved pharmacokinetic properties and in vivo efficacy in a melanoma angiogenesis model", BIOCONJUGATE CHEMISTRY,, vol. 17, no. 1, 1 January 2006 (2006-01-01), pages 179-188, XP002497163, DOI: 10.1021/BC050237Q [retrieved on 2005-12-03]**
• **BRICKELMAIER MARGOT ET AL: "Cytotoxicity of combinations of IFN-beta and chemotherapeutic drugs.", August 2002 (2002-08), JOURNAL OF INTERFERON & CYTOKINE RESEARCH : THE OFFICIAL JOURNAL OF THE INTERNATIONAL SOCIETY FOR INTERFERON AND CYTOKINE RESEARCH AUG 2002, VOL. 22, NR. 8, PAGE(S) 873 - 880, XP002720379, ISSN: 1079-9907 * page 877, column 2 * * page 874, column 2 - page 877, paragraph 1 ***
• **SEN S ET AL: "Heterogeneous interaction of interferon-beta and taxol in human ovarian cancer cells in vitro", EUROPEAN JOURNAL OF CANCER, PERGAMON PRESS, OXFORD, GB, vol. 30, no. 6, 1 January 1994 (1994-01-01), pages 892-893, XP022643218, ISSN: 0959-8049, DOI: 10.1016/0959-8049(94)90316-6 [retrieved on 1994-01-01]**
• **NICOLINI ET AL: "Metastatic breast cancer: an updating", BIOMEDICINE AND PHARMACOTHERAPY, ELSEVIER, FR, vol. 60, no. 9, 1 November 2006 (2006-11-01), pages 548-556, XP005720962, ISSN: 0753-3322, DOI: 10.1016/J.BIOPHA.2006.07.086**

EP 2 621 516 B1

**(Cont. next page)**

- CAMPISI C ET AL: "COMPLETE RESOLUTION OF BREAST CANCER BONE METASTASIS THROUGH THE USE OF BET-INTERFERON AND TAMOXIFEN", EUROPEAN JOURNAL OF GYNAECOLOGICAL ONCOLOGY, SEMES, PADOVA, IT, vol. 14, no. 6, 1 January 1993 (1993-01-01), pages 479-483, XP000918023, ISSN: 0392-2936

**Description**

**TECHNICAL FIELD**

This description relates to the use of a polymer-conjugated interferon-beta as monotherapy or in combination with other therapies to treat cancer.

**BACKGROUND**

In humans, cancers become established after a primary genetic event by a number of mechanisms that include, but are not limited to, increased cellular metabolism and growth rate, stimulation of angiogenesis thereby increasing blood supply to the tumor, and disregulation of signaling pathways and tumor suppressors. Moreover, tumors may become resistant to anti-cancer drugs by a number of mechanisms that include, but are not limited to, expulsion of the drug from the cell, occurrence of mutations that prevent binding of the drug to its target, and occurrence of additional mutations in genes and their protein products unrelated to the drug target. While cancer therapies typically use a combination of drugs to achieve efficacy, there remains a need to provide better therapies for a number of cancers for which long term prognosis is poor.

**SUMMARY**

Based on the disclosure that is contained herein, the present invention provides pegylated interferon-beta-1a for use in a method of treating breast carcinoma, wherein the treatment comprises administering said pegylated interferon-beta-1a to a subject in combination with a mitotic inhibitor.

In a related aspect, the present invention provides a mitotic inhibitor for use in a method of treating breast carcinoma, wherein the treatment comprises administering said mitotic inhibitor to a subject in combination with pegylated interferon-beta-1a.

In a further aspect, the invention provides pegylated interferon-beta-1a and mitotic inhibitor for use in a method of treating breast carcinoma, wherein the treatment comprises administering said pegylated interferon-beta-1a and mitotic inhibitor to a subject in combination.

The present invention and embodiments thereof are set out in the appended claims.

In one instance, a method of treating melanoma in a subject includes identifying a subject diagnosed with melanoma, administering an inhibitor of a protein kinase in the Ras-Raf-MEK-ERK pathway to the subject and administering an interferon beta to the subject. The inhibitor can be an inhibitor of MEK. The inhibitor can be administered in a pharmacologically-effective amount to treat melanoma. The inhibitor can be N-[(2R)-2,3-dihydroxypropoxy]-3,4-difluoro-2-[(2-fluoro-4-iodo-phenyl)amino]benzamide. The interferon beta can be interferon beta 1a. The interferon beta 1a can be pegylated interferon beta 1a. The interferon beta can be interferon beta 1b.

The interferon beta 1a can be pegylated interferon beta 1b. The IFN-beta or pegylated IFN-beta can be administered in a pharmacologically-effective amount to treat melanoma.

In another instance, a method of treating melanoma in a subject can include identifying a subject diagnosed with melanoma, administering an alkylating agent to the subject; and administering an interferon beta to the subject. The alkylating agent can be administered in a pharmacologically-effective amount to treat melanoma. The alkylating agent can be 4-methyl-5-oxo- 2,3,4,6,8-pentazabicyclo [4.3.0] nona-2,7,9-triene-9-carboxamide. The interferon beta can be interferon beta 1a. The interferon beta 1a can be pegylated interferon beta 1a. The interferon beta can be interferon beta 1b. The interferon beta 1a can be pegylated interferon beta 1b.

In another instance, a method of treating renal cell carcinoma in a subject can include identifying a subject diagnosed with renal cell carcinoma, administering an anti-VEGF antibody to the subject and administering an interferon beta to the subject. The anti-VEGF antibody can be administered in a pharmacologically-effective amount to treat renal cell carcinoma. The anti-VEGF antibody can be a humanized monoclonal anti-VEGF-A antibody. The interferon beta can be interferon beta 1a. The interferon beta 1a can be pegylated interferon beta 1a. The interferon beta can be interferon beta 1b. The interferon beta 1a can be pegylated interferon beta 1b. The IFN-beta or pegylated IFN-beta can be administered in a pharmacologically-effective amount to treat renal cell carcinoma.

In another instance, a method of treating renal cell carcinoma in a subject can include identifying a subject diagnosed with renal cell carcinoma, administering an inhibitor of mTOR to the subject and administering an interferon beta to the subject. The inhibitor of mTOR can be administered in a pharmacologically-effective amount to treat renal cell carcinoma. The inhibitor of mTOR can be temsirolimus. The interferon beta can be interferon beta 1a. The interferon beta 1a can be pegylated interferon beta 1a. The interferon beta can be interferon beta 1b. The interferon beta 1a can be pegylated interferon beta 1b.

In another instance, a method of treating renal cell carcinoma in a subject can include identifying a subject diagnosed with renal cell carcinoma, administering an inhibitor of Raf-1 to the subject and administering an interferon beta to the subject. The inhibitor of Raf-1 can be administered in a pharmacologically-effective amount to treat renal cell carcinoma. The inhibitor of Raf-1 can be 4-[4-[[4-chloro-3-(trifluoromethyl)phenyl]carbamoylamino]phenoxy]-N-methyl-pyridine-2-carboxamide. The interferon beta can be interferon beta 1a. The interferon beta 1a can be pegylated interferon beta 1a. The interferon beta can be interferon beta 1b. The interferon beta 1a can be pegylated interferon beta 1b.

In another instance, a method of treating colon carcinoma in a subject can include identifying a subject diagnosed with colon carcinoma, administering an anti-VEGF antibody to the subject and administering an interferon beta to the subject. The anti-VEGF antibody can be administered in a pharmacologically-effective amount to treat colon carcinoma. The anti-VEGF antibody can be a humanized monoclonal anti-VEGF-A antibody. The interferon beta can be interferon beta 1a. The interferon beta 1a can be pegylated interferon beta 1a. The interferon beta can be interferon beta 1b. The interferon beta 1a can be pegylated interferon beta 1b. The IFN-beta or pegylated IFN-beta can be administered in a pharmacologically-effective amount to treat colon carcinoma.

In another instance, a method of treating breast carcinoma in a subject can include identifying a subject diagnosed with breast carcinoma, administering a mitotic inhibitor to the subject and administering an interferon beta to the subject. The mitotic inhibitor can be administered in a pharmacologically-effective amount to treat breast carcinoma. The mitotic inhibitor can be paclitaxel. The interferon beta can be interferon beta 1a. The interferon beta 1a can be pegylated interferon beta 1a. The interferon beta can be interferon beta 1b. The interferon beta 1a can be pegylated interferon beta 1b. The IFN-beta or pegylated IFN-beta can be administered in a pharmacologically-effective amount to treat breast carcinoma.

In another instance, a method of treating breast carcinoma in a subject can include identifying a subject diagnosed with breast carcinoma and administering an interferon beta to the subject. The interferon beta can be interferon beta 1a. The interferon beta 1a can be pegylated interferon beta 1a. The interferon beta can be interferon beta 1b. The interferon beta 1a can be pegylated interferon beta 1b.

In another instance, a method of treating melanoma in a subject can include identifying a subject diagnosed with melanoma and administering an interferon beta to the subject. The interferon beta can be interferon beta 1a. The interferon beta 1a can be pegylated interferon beta 1a. The interferon beta can be interferon beta 1b. The interferon beta 1a can be pegylated interferon beta 1b.

In another instance, a method of treating renal cell carcinoma in a subject can include identifying a subject diagnosed with renal cell carcinoma and administering an interferon beta to the subject. The interferon beta can be interferon beta 1a. The interferon beta 1a can be pegylated interferon beta 1a. The interferon beta can be interferon beta 1b. The interferon beta 1a can be pegylated interferon beta 1b.

In another instance, a method of treating colon carcinoma in a subject can include identifying a subject diagnosed with colon carcinoma and administering an interferon beta to the subject. The interferon beta can be interferon beta 1a. The interferon beta 1a can be pegylated interferon beta 1a. The interferon beta can be interferon beta 1b. The interferon beta 1a can be pegylated interferon beta 1b.

Other features will become apparent from the following detailed description, taken in conjunction with the accompanying drawings, which illustrate by way of example, the features of the various embodiments.

**BRIEF DESCRIPTION OF THE DRAWINGS**

FIG. 1 is a graph that illustrates the effects of control, IFN-beta-1a, and pegylated IFN-beta-1a on the formation of tumor induced blood vessels in nude mice inoculated with $2X10^6$ SK-MEL-1 human melanoma cells.

FIG. 2 is a graph that illustrates the effects of different doses of the MEK inhibitor PD 325901 on SK-MEL-1 tumor volume in nude mice inoculated with SK-MEL-1 human melanoma cells.

FIG. 3 is a graph that illustrates the effects of vehicle controls, PD325901, pegylated Interferon-beta-1a, pegylated Interferon-beta-1a and PD325901 on SK-MEL-1 tumor volume in nude mice inoculated with SK-MEL-1 human melanoma cells.

FIG. 4 is a western blot illustrating the effects of vehicle controls, PD325901, pegylated Interferon-beta-1a, pegylated Interferon-beta-1a and PD325901 on ERK phosphorylation in SK-MEL-1 tumors.

FIG. 5 is a graph that illustrates tumor volume in nude mice of human A-375 melanoma tumors after treatment with vehicle controls, pegylated Interferon-beta-1a alone, temozolomide alone, and pegylated Interferon-beta-1a in combination with temozolomide.

FIG. 6A is a graph that illustrates tumor volume in SCID mice of human SN12C renal carcinoma tumors after treatment with vehicle controls, 0.8 mg/kg or 1.6 mg/kg pegylated Interferon-beta-1a. FIG. 6B is a graph that illustrates %T/C in SCID mice of human SN12C renal carcinoma tumors after treatment with vehicle controls, 0.8 mg/kg or 1.6 mg/kg pegylated Interferon-beta-1a.

FIG. 7A is a graph that illustrates tumor volume in SCID mice of human SN12C renal carcinoma tumors after treatment with vehicle controls, bevacizumab alone, pegylated Interferon-beta-1a alone, and a combination of bevacizumab and pegylated Interferon-beta-1a. FIG. 7B is a graph that illustrates %T/C in SCID mice of human SN12C renal carcinoma tumors after treatment with vehicle controls, bevacizumab alone, pegylated Interferon-beta-1a alone, and a combination of bevacizumab and pegylated Interferon-beta-1 a.

FIG. 8A is a graph that illustrates tumor volume in SCID mice of human SN12C renal carcinoma tumors after treatment with vehicle controls, sorafenib alone, pegylated interferon beta 1a alone, and a combination of sorafenib and pegylated interferon beta 1a in a concurrent dosing schedule. FIG. 8B is a graph that illustrates tumor volume in SCID mice of human SN12C renal carcinoma tumors after treatment with vehicle controls, sorafenib alone, pegylated interferon beta 1a alone, and a combination of sorafenib and pegylated interferon beta 1a in a sequential dosing schedule.

FIG. 9A is a graph that illustrates tumor volume in SCID mice of human SN12C renal carcinoma tumors after treatment with vehicle controls, temsirolimus alone, pegylated Interferon-beta-1a alone, and a combination of temsirolimus and pegylated Interferon-beta-1a. FIG 9B is a graph that illustrates %T/C in SCID mice of human SN12C renal carcinoma tumors after treatment with vehicle controls, temsirolimus alone, pegylated Interferon-beta-1a alone and a combination of temsirolimus and pegylated Interferon-beta-1 a.

FIG. 10 is a graph that illustrates tumor volume in nude mice of human SW-620 colon carcinoma tumors after treatment with vehicle control, and pegylated IFN-beta-1a at different doses.

FIG. 11 is a graph that illustrates tumor volume in nude mice of human SW-620 colon carcinoma tumors after treatment with vehicle controls, avastin (bevacizumab) alone, irinotecan alone, pegylated Interferon-beta-1a alone, a combination of avastin and pegylated Interferon-beta-1a, and a combination of irinotecan and pegylated Interferon-beta-1a.

FIG. 12 is a graph that illustrates tumor volume in SCID mice of human MDA-MB-231 breast carcinoma tumors treated with vehicle controls and paclitaxel.

FIG. 13A is a graph that illustrates tumor volume in SCID mice of human MDA-MB-231 breast carcinoma tumors treated with vehicle controls and pegylated IFN-beta-1a at different doses. FIG. 13B is a graph that illustrates %T/C in SCID mice of human MDA-MB-231 breast carcinoma tumors treated with vehicle controls and pegylated Interferon-beta-1a at different doses. FIG. 13C is a graph that illustrates tumor volume in nude mice of human MDA-MB-468 breast carcinoma tumors treated with vehicle controls, IFN-beta-1a, or pegylated IFN-beta-1a.

FIG. 14A is a graph that illustrates tumor volume in SCID mice of human MDA-MB-231 breast carcinoma tumors treated with vehicle control, pegylated IFN-beta-1a alone, paclitaxel alone, and a combination of pegylated IFN-beta-1a and paclitaxel. FIG. 14B is a graph that illustrates %T/C in SCID mice of human MDA-MB-231 breast carcinoma tumors treated with vehicle control, pegylated IFN-beta-1a alone, paclitaxel alone, and a combination pegylated IFN-beta-1a and paclitaxel. The dose of pegylated interferon beta 1a used in FIG. 14A and 14B was 0.8 mg/kg.

FIG. 15A is a graph that illustrates tumor volume in SCID mice of human MDA-MB-231 breast carcinoma tumors treated with vehicle control, pegylated IFN-beta-1a alone, paclitaxel alone, and a combination of pegylated IFN-beta-1a and paclitaxel. FIG. 15B is a graph that illustrates %T/C in SCID mice of human MDA-MB-231 breast carcinoma tumors treated with vehicle control, pegylated IFN-beta-1a alone, paclitaxel alone, and a combination pegylated IFN-beta-1a and paclitaxel. The concentration of pegylated interferon beta 1a used in FIG. 15A and 15B was 1.6 mg/kg.

FIG. 16 is a graph that illustrates tumor volume in nude mice of human WM-266-4 melanoma tumors treated with pegylated IFN-beta-1a at 0.1 mg/kg, 0.2 mg/kg, 0.4 mg/kg, and 0.8 mg/kg.

FIGS. 17A-C are graphs that illustrate tumor volume in nude mice of human WM-266-4 melanoma tumors treated with vehicle control, 0.4mg/kg, 0.8 mg/kg, or 1.6 mg/kg pegylated interferon-beta-1a once a week (QW), twice a week (BIW), or three times a week (TIW).

FIG. 18 is a graph that illustrates tumor volume in nude mice of large human WM-266-4 melanoma tumors treated with pegylated IFN-beta-1a at 0.8 mg/kg and 1.6 mg/kg.

FIG. 19A is a western blot that illustrates the effect on caspase activation and PARP cleavage in WM-266-4 melanoma cells treated with pegylated interferon-beta-1a for 48 hours. FIG. 19B-C are graphs that illustrate fold caspase 3 and PARP induction, respectively, in WM-266-4 melanoma cells treated with pegylated interferon-beta-1a for 48 hours.

## DETAILED DESCRIPTION

Interferon-beta is currently being used for the treatment of multiple sclerosis. For example, interferon-beta-1a is marketed in the United States under the trade names of AVONEX™ (Biogen Idec MA Inc.) and REBIF™ (EMD Serono) and interferon-beta-1b is marketed in the United States as BETASERON™ (Berlex) and EXTAVIA™ (Novartis).

The term "interferon" or "IFN" as used herein means the family of highly homologous species-specific proteins that inhibit viral replication and cellular proliferation and modulate immune responses. Human interferons are grouped into two classes; Type I, including alpha and beta-interferon, and Type II, which is represented by gamma-interferon only. Recombinant forms of each group have been developed and are commercially available. Subtypes in each group are based on antigenic/structural characteristics. An example of interferon used herein is a human interferon-beta that is glycosylated at residue 80 (Asn 80) and that is derived via recombinant DNA technologies. A further example of interferon used herein is pegylated Interferon beta-1a which is modified at the N-terminal $\alpha$-amino group with a single, linear 20 kDa mPEG-O-2-methylpropionaldehyde group.

This glycosylated interferon-beta is also known as "interferon-beta-1a" or "IFN-beta-1a" or "interferon beta 1a," all used interchangeably. The term "interferon-beta-1a" is also meant to encompass mutants thereof, provided that such mutants are also glycosylated at residue 80 (Asn 80). Recombinant DNA methods for producing proteins, including interferons are known. See for example, U.S. Pat. Nos. 4,399,216, 5,149,636, 5,179,017 (Axel et al) and U.S. Pat. No. 4,470,461 (Kaufman). Another example of interferon that can be used is interferon beta-1b. Interferon beta-1b can be produced in *E. coli* bacteria using a modified human gene sequence that contains a genetically engineered cysteine-to-serine substitution at amino acid position 17 and can be non-glycosylated.

Mutants of interferon-beta-1a may be used. Mutations are developed using conventional methods of directed mutagenesis, known to those of ordinary skill in the art and can include functionally equivalent interferon-beta-1a polynucleotides that encode for functionally equivalent interferon-beta-1a polypeptides.

The construction of recombinant DNA plasmids containing sequences encoding at least part of human fibroblast interferon and the expression of a polypeptide having immunological or biological activity of human fibroblast interferon is also contemplated. The construction of hybrid beta-interferon genes containing combinations of different subtype sequences can be accomplished by techniques known to those of skill in the art.

The interferon-beta protein may be conjugated to polyalkylene glycol residues of C1-C4 alkyl polyalkylene glycols preferably polyethylene glycol (PEG), or poly(oxy)alkylene glycol residues of such glycols. Thus, the polymer to which the protein is attached can be a homopolymer of polyethylene glycol (PEG) or is a polyoxyethylated polyol, provided in all cases that the polymer is soluble in water at room temperature. Non-limiting examples of such polymers include polyalkylene oxide homopolymers such as PEG or polypropylene glycols, polyoxyethylenated glycols, copolymers thereof and block copolymers thereof, provided that the water solubility of the block copolymer is maintained. Examples of polyoxyethylated polyols include, for example, polyoxyethylated glycerol, polyoxyethylated sorbitol, polyoxyethylated glucose, or the like. The glycerol backbone of polyoxyethylated glycerol is the same backbone occurring naturally in, for example, animals and humans in mono-, di-, and triglycerides. Therefore, this branching would not necessarily be seen as a foreign agent in the body.

As an alternative to polyalkylene oxides, dextran, polyvinyl pyrrolidones, polyacrylamides, polyvinyl alcohols, carbohydrate-based polymers and the like may be used. Those of ordinary skill in the art will recognize that the foregoing list is merely illustrative and that all polymer materials having the qualities described herein are contemplated. The polymer need not have any particular molecular weight, but it is preferred that the molecular weight be between about 300 and 100,000, more preferably between 10,000 and 40,000. In particular, sizes of 20,000 or more are best at preventing protein loss due to filtration in the kidneys.

Polyalkylene glycol derivatization has a number of advantageous properties in the formulation of polymer-interferon-beta 1a conjugates, as associated with the following properties of polyalkylene glycol derivatives: improvement of aqueous solubility, while at the same time eliciting no antigenic or immunogenic response; high degrees of biocompatibility; absence of in vivo biodegradation of the polyalkylene glycol derivatives; and ease of excretion by living organisms. An example of a pegylated interferon-beta-1a that can be used in the compositions described herein can include interferon-beta-1a modified at the N-terminal $\alpha$-amino group with a single linear 20 kDa mPEG-O-2-methylpropionaldehyde group (See Baker DP et al 2006, Bioconjugate Chem. 17, 179-188). Conjugation of interferon-beta to PEG including mPEG-propionaldehyde is further described in U.S. Patent No. 7,446,173 and U.S. Application Publication No. 20050107277.

The compositions described herein can be formulated as aqueous pharmaceutical compositions or in lyopholized form. A stable composition of interferon-beta exhibits little or no signs of any one or more of aggregation, fragmentation, deamidation, oxidation, or change in biological activity over an extended period of time, e.g., 12 months, 24 months, 36 months or longer. For example, in one embodiment, less than 10% of the composition is aggregated, fragmented, or oxidized. Aggregation, precipitation, and/or denaturation can be assessed by known methods, such as visual examination of color and/or clarity, or by UV light scattering or size exclusion chromatography. The ability of the protein to retain its biological activity can be assessed by detecting and quantifying chemically altered forms of the protein. Size modification (e.g., clipping) can be evaluated using size exclusion chromatography, SDS-PAGE and/or matrix-assisted laser desorption ionization/time-of-flight mass spectrometry (MALDI/TOF MS), or peptide mapping of endoproteinase-treated protein, for example. Other types of chemical alteration include charge alteration (e.g., occurring as a result of deamidation), which can be evaluated by ion-exchange chromatography, for example. A protein retains its biological activity in a pharmaceutical formulation, if the biological activity of the protein at a given time is within about 10% of the biological activity exhibited at the time the pharmaceutical formulation was prepared as determined in an assay.

Interferon-beta or interferon-beta-1a or pegylated interferon-beta-1a, for example, can be provided in a buffered solution at a concentration of about 0.1 mg/ml to about 5 mg/ml, of about 0.1 mg/ml to about 1 mg/ml, of about 0.1 mg/ml to about 0.5 mg/ml, of about 0.1 mg/ml to about 0.3 mg/ml, or of about 0.1 mg/ml to about 0.2 mg/ml. Interferon-beta-1a can be provided in a buffered solution at a concentration of 0.25 mg/ml. The composition can be stored at 2-25°C, at 5°C, at 10°C, at 15°C, at 20°C or at 25°C. The composition can be stable at room temperature for 1, 2, 3, 4, or 5 days or more. Room temperature can be about 18°C, 19°C, 20°C, 21°C, 22°C, 23°C, 24°C or 25°C. The composition can be stored at a first low temperature, for example, less than 18°C, or from about freezing but at or below 15°C, 10°C or 4°C and can be stored at second higher temperature, for example, without refrigeration or at room temperature, for about 1-5 days.

Pharmaceutical compositions are sterile and stable under the conditions of manufacture and storage. A pharmaceutical composition can also be tested to insure it meets regulatory and industry standards for administration.

Exemplary conditions which can be treated with interferon include, but are not limited to, cell proliferation disorders including cancers, multiple sclerosis, and viral infections. Without limitation, treatment with interferon may be used to treat conditions which would benefit from inhibiting the replication of interferon-sensitive viruses. A cancer can include an adrenocortical carcinoma, anal cancer, bladder cancer, brain tumor, glioma, breast carcinoma, carcinoid tumor, cervical cancer, colon carcinoma, endometrial cancer, esophageal cancer, extrahepatic bile duct cancer, Ewings tumor, extracranial germ cell tumor, eye cancer, gall bladder cancer, gastric cancer, germ cell tumor, gestational trophoblastic tumor, head and neck cancer, hypopharyngeal cancer, islet cell carcinoma, kidney cancer, laryngeal cancer, leukemia, lip and oral cavity cancer, liver cancer, lung cancer, lymphoma, melanoma, mesothelioma, merkel cell carcinoma, metastatic squamous head and neck cancer, myeloma, neoplasm, nasopharyngeal cancer, neuroblastoma, oral cancer, oropharyngeal cancer, osteosarcoma, ovarian cancer, pancreatic cancer, sinus and nasal cancer, parathyroid cancer, penile cancer, pheochromocytoma cancer, pituitary cancer, plasma cell neoplasm, prostate cancer, rhabdomyosarcoma, rectal cancer, renal cell carcinoma, salivary gland cancer, skin cancer, Kaposi's sarcoma, T-cell lymphoma, soft tissue sarcoma, stomach cancer, testicular cancer, thymoma, thyroid cancer, urethral cancer, uterine cancer, vaginal cancer, vulvar cancer, or Wilms' tumor.

The IFN-beta or pegylated IFN-beta can be administered in a pharmacologically-effective amount to treat any of the conditions described above. The term "pharmacologically-effective amount" means the amount of a drug or pharmaceutical agent that will elicit the biological or medical response of a tissue, system, animal or human that is being sought by a researcher or clinician. It is an amount that is sufficient to significantly affect a positive clinical response while maintaining diminished levels of side effects. The amount of pegylated IFN-beta for example, which may be administered to a subject in need thereof is in the range of 0.01-1000 $\mu$g/kg, or more preferably 0.01-100 $\mu$g/kg. Interferon-beta-1a or pegylated interferon-beta-1a can be provided in a buffered solution at a concentration range of 0.1-5 mg/mL, or more preferably 0.25-1 mg/mL and can be administered in single or divided doses. The term "subject" refers to any animal, e.g., a mammal, human, or non-human. Exemplary subjects include, but are not limited to, humans, non-human primates, mice, rats, guinea pigs, cattle, sheep, goats, pigs, dogs, cats, birds, deer, elk, rabbit, reindeer, deer, and horses.

Administration of the described dosages may be every other day, but preferably occurs once a week or once every other week. Doses can be administered over at least a 24 week period by injection. The dosage regimen utilizing the composition described herein is selected in accordance with a variety of factors including type, species, age, weight, sex, and medical condition of the patient; the severity of the condition to be treated; the route of administration; the renal and hepatic function of the patient; and the particular compound or salt thereof employed. The activity of the interferon-beta and sensitivity of the patient to side effects are also considered. An ordinarily skilled physician or veterinarian can readily determine and prescribe the effective amount of the drug required to prevent, counter, or arrest the progress of the condition.

In yet another embodiment, the composition is suitable for subcutaneous or intramuscular administration. In even another embodiment, the composition is suitable for IV administration. The compositions described herein can be administered by a parenteral mode (e.g., subcutaneous, intraperitoneal, or intramuscular injection). The phrases "parenteral administration" and "administered parenterally" as used herein mean modes of administration other than enteral and topical administration, usually by injection, and include, subcutaneous or intramuscular administration, as well as intravenous, intracapsular, intraorbital, intracardiac, intradermal, intraperitoneal, transtracheal, subcuticular, subcapsular, subarachnoid, intraspinal, epidural, and intrasternal injection and infusion. Administration of the dose can be intravenous, subcutaneous, intramuscular, or any other acceptable systemic method. Based on the judgment of the attending clinician, the amount of drug administered and the treatment regimen used will, of course, be dependent on the age, sex, and medical history of the patient being treated, the neutrophil count (e.g., the severity of the neutropenia), the severity of the specific disease condition, and the tolerance of the patient to the treatment as evidenced by local toxicity and by systemic side-effects.

Parental injectable administration is generally used for subcutaneous, intramuscular, or intravenous injections and infusions. For example, a subcutaneous injection can be used to deliver a range of 0.01-1000 µg/kg, or more preferably 0.01-100 µg/kg. Interferon-beta-1a or pegylated interferon-beta-1a can be provided in a buffered solution at a concentration range of 0.1-5 mg/mL, or more preferably 0.25-1 mg/mL. Additionally, one approach for parenteral administration employs the implantation of a slow-release or sustained-released system, which assures that a constant level of dosage is maintained, according to U.S. Pat. No. 3,710,795.

Lyophilized compositions for injections can, for example, be prepared by dissolving, dispersing, etc. The active compound is dissolved in or mixed with a pharmaceutically-pure solvent such as, for example, water, saline, aqueous dextrose, glycerol, ethanol, and the like, to form the injectable solution or suspension. Additionally, solid/lyophilized forms suitable for dissolving in liquid prior to injection can be formulated. Injectable compositions are preferably aqueous isotonic solutions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically-valuable substances.

Pharmaceutical compositions can be administered with medical devices. For example, pharmaceutical compositions can be administered with a needleless hypodermic injection device, such as the devices disclosed in U.S. Pat. Nos. 5,399,163, 5,383,851, 5,312,335, 5,064,413, 4,941,880, 4,790,824, or 4,596,556. Examples of well-known implants and modules include: U.S. Pat. No. 4,487,603, which discloses an implantable micro-infusion pump for dispensing medication at a controlled rate; U.S. Pat. No. 4,486,194, which discloses a therapeutic device for administering medicants through the skin; U.S. Pat. No. 4,447,233, which discloses a medication infusion pump for delivering medication at a precise infusion rate; U.S. Pat. No. 4,447,224, which discloses a variable flow implantable infusion apparatus for continuous drug delivery; U.S. Pat. No. 4,439,196, which discloses an osmotic drug delivery system having multi-chamber compartments; and U.S. Pat. No. 4,475,196, which discloses an osmotic drug delivery system. The therapeutic composition can also be in the form of a biodegradable or nonbiodegradable sustained release formulation for subcutaneous or intramuscular administration. See, e.g., U.S. Pat. Nos. 3,773,919 and 4,767,628 and PCT Application No. WO 94/15587. Continuous administration can also be achieved using an implantable or external pump. The administration can also be conducted intermittently, e.g., single daily injection, or continuously at a low dose, e.g., sustained release formulation. The delivery device can be modified to be optimally suited for administration of interferon-beta. For example, a syringe can be siliconized to an extent that is optimal for storage and delivery of interferon-beta. Of course, many other such implants, delivery systems, and modules are also known.

In another instance, the IFN-beta or pegylated IFN-beta is administered in combination with a second therapeutic agent in a pharmacologically-effective amount to treat any of the conditions described above. A method of inhibiting cancer proliferation, or treating cancer in a subject, can include identifying a subject diagnosed with a cancer. Identifying a subject diagnosed with cancer can include self identification, identification by diagnosis, identification by referral from a physician or identification by referral from an organization. Identification by diagnosis can include biopsies, imaging tests, laboratory tests, genomic tests or palpations. A referring organization can be a clinic, hospital, agency or support group.

Inhibiting cancer proliferation can include slowing down the rate at which the cancer cells undergo mitosis. Treating cancer in a subject can include inhibiting cancer proliferation and improving the overall medical condition of the subject.

The subject can be treated with pegylated interferon-beta-1a alone as a monotherapy or in combination with other suitable drugs as described herein depending on the type of cancer. The drugs can be administered sequentially which can be in a predetermined order or administered concurrently. A method of treating cancer in a subject can include determining a sufficient time after administering pegylated interferon-beta-1a alone or in combination if a sign or symptom of a particular cancer is inhibited to determine if the treatment is effective. A method of inhibiting cancer proliferation in a subject can include determining a sufficient time after administering pegylated interferon-beta-1a alone or in combination if a sign or symptom of a particular cancer is inhibited to determine if the treatment is effective. A sufficient time can be at least 1 day, at least 1 week, at least 2 weeks, at least 3 weeks, at least 1 month, at least 2 months, at least 3 months, at least 6 months, at least 1 year, at least 2 years, at least 5 years or more than 5 years later. Time can be measured from the time when either pegylated interferon-beta-1a alone or in combination with a second drug or a second drug is administered.

A sign or symptom of cancer can include tumor size. Tumor size can be measured by measuring the minor diameter of the tumor, the major diameter of the tumor, or calculating the volume of the tumor. Tumor size can be determined by palpations, x-rays, computed tomography scans, magnetic resonance imaging, positron emission tomography or bone scans, among others.

A cellular change can be a sign or symptom of a cancer. Cellular changes can be determined by taking a biopsy of the tumor and using histological techniques to examine it. A cellular change can include an increased rate of cell proliferation, a decreased rate of cell death or a decreased need for adhesion. A sign or symptom can include altered protein expression. This can include misregulation or disregulation of the expression of a protein. The altered protein expression can include underexpression, overexpression, or a change in post-translational modification. The altered protein expression can include a presence of the protein in a region where the protein is not normally present or an absence of the protein in a region where the protein is normally present. The altered protein expression can also include changes in timing related to the protein. For example, the protein is present at a developmental, cell cycle or signal transduction stage in which it is normally not present or is absent when it is normally present. Altered protein expression can result in the activity of a protein being modified. The protein may be more active or less active. An activity may be absent, a new activity may develop or the specificity of an activity can change. The protein may also be activated or inactivated at an incorrect time in a developmental, cell cycle or signal transduction stage.

A sign or symptom of cancer can include attributes of the skin, including attributes of a mole, blemish, freckle, sore, lump, mark or sore. The attributes can include the shape, color or size of the mole, blemish, freckle, lump, mark or sore. The shape can be asymmetrical, irregular, ragged, notched or blurred. The color can be uneven and include black, brown, red, pink, blue or white. The size can be greater than 4 millimeters, greater than 5 millimeters or greater than 6 millimeters. The number of moles, blemishes, freckles, lumps, marks or sores can also be a sign or symptom of melanoma. The duration the mole, blemish, freckle, lump, mark or sore has been present can be a sign or symptom of melanoma. Another sign or symptom can be a mole, blemish, freckle, lump, mark or sore that does not heal or that increases in size and severity. Changes in skin sensation, for example tenderness, itchiness or pain, can be a sign or symptom of melanoma. Additionally, changes in the surface of the mole, blemish, freckle, lump, mark or sore can be a sign or symptom. For example, the change can include scaliness, oozing, bleeding or other appearance change.

Inhibited can mean that the sign or symptom is decreased or the number of signs or symptoms is decreased. A decrease in the number of signs or symptoms can include the total number of occurrences of different signs or symptoms or the total number of occurrences of the same sign or symptom. For example, the tumor volume can decrease or the number of tumors could decrease. Another example can include the sign of an underexpressed protein. A decrease in this sign could be an increase in expression. In a similar manner, a decrease in the sign including loss of protein activity could be an increase in protein activity.

Melanoma

A cancer can include melanoma. The melanoma can be a superficial spreading melanoma, a nodular melanoma, a lentigo maligna melanoma or an acral lentginous melanoma. The melanoma can include a BRAF mutant. The BRAF mutant can be a BRAF V600E mutant.

A method of inhibiting cancer proliferation, or treating cancer which can include melanoma in a subject, can include administering an inhibitor of a protein kinase in combination with IFN-beta or pegylated IFN-beta to a subject. The protein kinase can be in the Ras-Raf-MEK-ERK pathway. The inhibitor of a protein kinase in the Ras-Raf-MEK-ERK pathway can be an inhibitor of EGFR, Ras, B-Raf, MEK or ERK. The inhibitor can be cetuximab, erlotinib, trastuzumab, imatinib or oblimersen. The inhibitor can be a farnesyltransferase inhibitor or a farnesylthiosalicylic acid. The inhibitor can also be GW5074 (Glaxo Wellcome, Inc.), BAY 43-9006 (Bayer AG), Ro 09-2210 (Roche Products Limited), L-783277 (Merck), PD 169316 (Calbiochem), SB 203580 (Calbiochem), U 0126 (Calbiochem), PD 98059 (Calbiochem), PD 184352 (United States Biological), PD 0325901 or AZD 8330 (AstraZeneca), FR180204 (Calbiochem) or calpeptin. The inhibitor can be a N-[(2R)-2,3-dihydroxypropoxy]-3,4-difluoro-2-[(2-fluoro-4-iodo-phenyl)amino]benzamide (PD 0325901).

The dose of the inhibitor of a protein kinase administered to a subject can be less than 10 $\mu$g, less than 25 $\mu$g, less than 50 $\mu$g, less than 75 $\mu$g, less than 0.10 mg, less than 0.25 mg, less than 0.5 mg, less than 1 mg, less than 2.5 mg, less than 5 mg, less than 10 mg, less than 15 mg, less than 20 mg, less than 50 mg, less than 75 mg, less than 100 mg, less than 200 mg, less than 300 mg, less than 400 mg, less than 500 mg, less than 750 mg, less than 1 g, less than 2 g or less than 5 g.

In another instance, a method of inhibiting cancer proliferation, or treating cancer which can include melanoma in a subject, can include administering an alkylating or methylating DNA agent in combination with IFN-beta or pegylated IFN-beta to a subject. An alkylating agent can be an agent that attaches an alkyl group ($C_nH_{2n+1}$) to DNA. The alkyl group is attached to the guanine base of DNA, at the number 7 nitrogen atom of the imidazole ring. The alkylating agent can include 4-methyl-5-oxo- 2,3,4,6,8-pentazabicyclo [4.3.0] nona-2,7,9-triene- 9-carboxamide (temozolomide, also known as TEMODAR®).

The dose of an alkylating or methylating DNA agent, for example temozolomide administered to a subject can be between 50 mg/m$^2$ to 200 mg/m$^2$. Temozolomide can be administered to a subject at a dose of about 50 mg/m$^2$, 75 mg/m$^2$, 90 mg/m$^2$, 100 mg/m$^2$, 135 mg/m$^2$, 175 mg/m$^2$, 200 mg/m$^2$ or more over a period of time. Temozolomide can be administered once daily for 5 days, 10 days, 15 days, 20 days, 25 days, 30 days, 35 days, 40 days or 50 days or longer. Temozolomide can be administered in tablet form or through injection. Temozolomide can be administered in a 28 day cycle for one cycle, 2 cycles, 3 cycles, 4 cycles, 5 cycles, 6 cycles or more as needed. Temozolomide can be administered intravenously over 60 minutes or less, 90 minutes, 120 minutes, 150 minutes or more. Temozolomide can be administered for an additional maintenance phase following the initial cycle of treatment. Other suitable doses and length of administration of temozolomide for use in combination with IFN-beta or pegylated IFN-beta can be determined by methods known to a person of skill in the art.

Renal cell carcinoma

A cancer can include a renal cell carcinoma. Renal cell carcinoma (also known as renal adenocarcinoma or hypernephromais) is a type of kidney cancer in which the cancerous cells are found in the lining of very small tubes (tubules) in the kidney. Renal cell carcinoma can include clear cell renal cell carcinoma, papillary renal cell carcinoma, chromophone renal cell carcinoma, collecting duct renal cell carcinoma, and unclassified renal cell carcinoma.

In a further instance, a method of inhibiting cancer proliferation, or treating cancer such as renal cell carcinoma in a subject, can include administering pegylated IFN-beta to a subject. In another instance, a method of inhibiting cancer proliferation, or treating cancer such as renal cell carcinoma in a subject, can include administering an anti-VEGF antibody in combination with IFN-beta or pegylated IFN-beta to a subject. The antibody can be a monoclonal or polyclonal antibody or fragments thereof. The antibody can be a humanized monoclonal antibody such as bevacizumab (Avastin™, Genentech/Roche).

The dose of an anti-VEGF antibody administered to a subject can be between 0.5 mg/kg to 20 mg/kg. For example, bevacizumab may be administered intravenously at a dose of 1 mg/kg, 2.5 mg/kg, 5 mg/kg, 7.5 mg/kg, 10 mg/kg, 12.5 mg/kg or 15 mg/kg for a period of time. The period time can include every week, every two weeks, every three weeks or every month. Other suitable doses and length of administration of bevacizumab for use in combination with IFN-beta or pegylated IFN-beta can be determined by methods known to a person of skill in the art.

In another instance, a method of inhibiting cancer proliferation, or treating cancer such as renal cell carcinoma in a subject, can include administering a mammalian target of rapamycin (mTOR) inhibitor in combination with IFN-beta or pegylated IFN-beta to a subject. mTOR inhibitors can include temsirolimus (Torisel™, Wyeth).

The dose of a mTOR inhibitor, for example temsirolimus administered to a subject can be between 10 mg to 60 mg, 15mg to 55 mg, 20 mg to 50 mg, 25 mg to 45 mg. Temsirolimus can be administered to a subject at a dose of 25 mg infused over a period of time. The period of time can be 10-120 minutes or 30-60 minutes once a week or several times a week, once every other week or several times every other week, or once a month or several times in a month. Other suitable doses and lengths of administration of temsirolimus for use in combination with IFN-beta or pegylated IFN-beta can be determined by methods known to a person of skill in the art.

In another embodiment, a method of inhibiting cancer proliferation, or treating cancer such as renal cell carcinoma in a subject, can include administering a serine/threonine or tyrosine protein kinase inhibitor in combination with IFN-beta or pegylated IFN-beta to a subject. A serine/threonine or tyrosine protein kinase inhibitor can include inhibitors of the Ras/Raf/Mek/Erk pathway such as an inhibitor of Raf-1. An inhibitor of Raf-1 can include 4-[4-[[4-chloro-3-(trifluoromethyl)phenyl]carbamoylamino]phenoxy]-N-methyl-pyridine-2-carboxamide (sorafenib, also known as NEXAVAR®).

The dose of a Raf-1 inhibitor, for example sorafenib administered to a subject can be between 200-800 mg. Sorafenib may be taken in tablet form at a dose of 200 mg, 400mg or 800 mg once a day, twice daily, three times daily, or four times daily. Other suitable doses and length of administration of sorafenib for use in combination with IFN-beta or pegylated IFN-beta can be determined by methods known to a person of skill in the art.

Colon carcinoma

A cancer can include colon carcinoma which is a cancer of the large intestine. Colon cancer can start out as small, noncancerous (benign) clumps of cells called adenomatous polyps which may develop into colon cancer. Rectal cancer is the cancer of the last several inches of the colon and can often times develop together with colon cancer (also known as colorectal cancer).

In a further instance, a method of inhibiting cancer proliferation, or treating cancer such as colon carcinoma in a subject, can include administering pegylated IFN-beta to a subject. In another instance, a method of inhibiting cancer proliferation, or treating cancer such as colon carcinoma in a subject, can include administering anti-VEGF antibody in combination with IFN-beta or pegylated IFN-beta to a subject. The antibody can be a monoclonal or polyclonal antibody or fragments thereof. The antibody can be a humanized monoclonal antibody such as bevacizumab (Avastin™, Genentech/Roche).

Breast carcinoma

Breast cancer is a cancer that starts in the tissues of the breast and can include ductal carcinoma and lobular carcinoma. In rare cases, breast cancer can start in other areas of the breast. Breast cancers can be sensitive to estrogen. Breast cancer can be HER2 positive. Current treatments for breast cancer include tamoxifen which blocks the effects of estrogen or targeted therapy which includes herceptin.

In a further instance, a method of inhibiting cancer proliferation, or treating cancer such as breast carcinoma in a subject, can include administering pegylated IFN-beta to a subject. In another instance, a method of inhibiting cancer proliferation, or treating cancer such as breast carcinoma in a subject, can include administering a mitotic inhibitor in combination with IFN-beta or pegylated IFN-beta to a subject. A mitotic inhibitor can include taxol (paclitaxel).

The doses of paclitaxel administered to a subject can be between 5 mg/m$^2$ to 200 mg/m$^2$. Paclitaxel can be administered intravenously to a subject at a dose of about 50 mg/m$^2$, 90 mg/m$^2$, 100 mg/m$^2$, 135 mg/m$^2$ or 175 mg/m$^2$ over a period of time. Paclitaxel can be administered over 1 hour, 2 hours, 3 hours, 4 hours, 5 hours, 10 hours, 15 hours, 20 hours or 24 hours or more every 1 week, 2, weeks, 3 weeks, month or more. Other suitable doses and length of administration of paclitaxel for use in combination with IFN-beta or pegylated IFN-beta can be determined by methods known to a person of skill in the art.

**EXAMPLE 1**

Anti-angiogenic effect of Pegylated Interferon-beta-1a

Athymic nude mice were inoculated with 2X10$^6$ SK-MEL-1 human melanoma cells (1 injection per flank, 1 injection per mouse) and tumors (100-200 cubic mm) were established. In a single dose study, groups of 4 mice carrying SK-MEL-1 human melanoma tumors received once on day 1 the following: vehicle control, 5 $\mu$g (1 MU) IFN-beta-1a or 10 $\mu$g (1 MU) pegylated IFN-beta-1a. The pegylated Interferon beta-1a as used throughout the examples described herein is modified at the N-terminal $\alpha$-amino group with a single, linear 20 kDa mPEG-O-2-methylpropionaldehyde group. In a multiple dose study, groups of 3 mice carrying SK-MEL-1 human melanoma tumors received the following: vehicle control once on day 1 only, 10 $\mu$g (1 MU) pegylated IFN-beta-1a once on day 1 only, vehicle control on days 1-9 or 5 $\mu$g (1 MU) IFN-beta-1a on days 1-9. Mice were then sacrificed on day 10 and the number of vessels entering the periphery of the tumor was counted. The assessor of neovascularisation was blinded as to treatment groups.

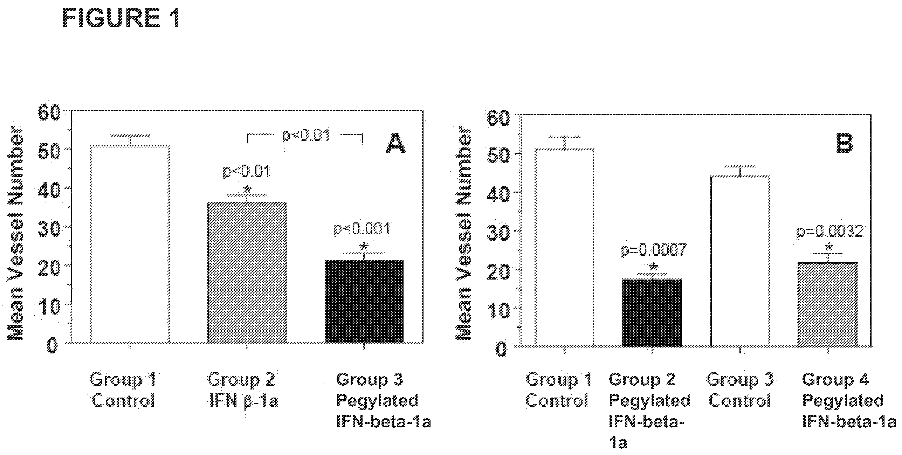

Results: The half-life of pegylated IFN-beta-1a in nude mice is approximately 10 hours. Figure 1 illustrates the effects of control, IFN-beta-1a, and pegylated IFN-beta-1a on the formation of tumor induced blood vessels in nude mice inoculated with SK-MEL-1 human melanoma cells. Pegylated IFN-beta-1a was significantly more effective at reducing formation of tumor induced blood vessels. In addition, pegylated IFN-beta-1a given once on day 1 was more effective than IFN-beta-1a given once on day 1. See Figure 1A. Pegylated IFN-beta-1a given once on day 1 was comparable (slightly superior) to IFN-beta-1a given daily on days 1-9. See Figure 1B. It is unlikely that the human protein acts on murine vessel cells since human IFN-beta-1a is not active on murine (L929) cells at concentrations 10-fold greater than required for full activity of murine IFN-beta (i.e. species specificity is required). Pegylated IFN-beta-1a is more likely to act on the tumor itself by either specifically inhibiting the production of pro-angiogenic factors or inhibiting the growth of the tumor and thereby indirectly inhibiting the production of pro-angiogenic factors.

Anti-angiogenic effect of the MEK inhibitor PD325901

Athymic nude mice were inoculated with 2X10$^6$ SK-MEL-1 human melanoma cells (1 injection per flank; 2 injections per mouse) and 100-200 cubic mm tumors were established. Mice (5 per group) were randomized and treated with 0, 1,5, 10 and 20 mg/kg PD 325901 in PEG400 PO (orally) once per day (QD) for 14 days. Tumors were measured every 3 days using calipers and volume was calculated for a prolate spheroid, defined as:

$$V = (pi*L*W^2)/6$$

where L equals the major diameter and W equals the minor diameter.

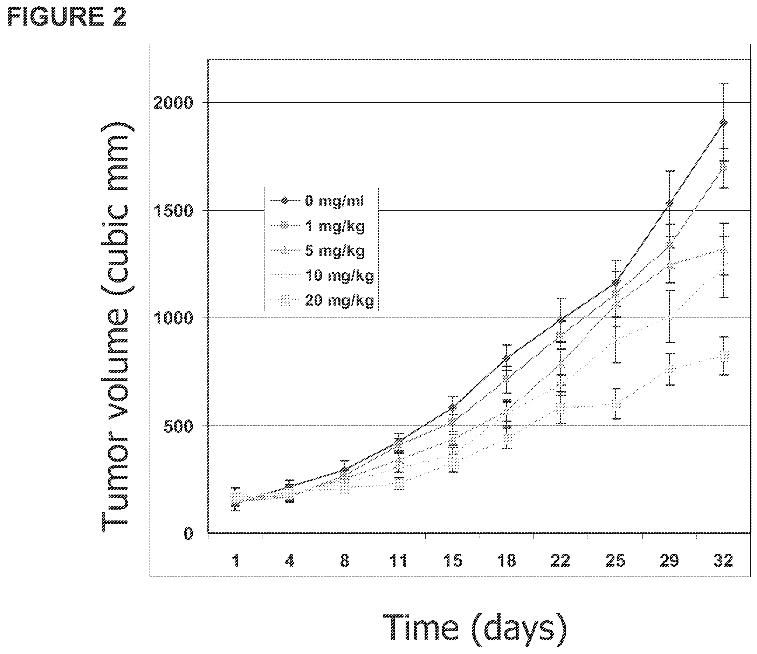

Results: Figure 2 illustrates the effects of different doses of PD 325901 on SK-MEL-1 tumor volume in nude mice inoculated with SK-MEL-1 human melanoma cells. From this experiment, the dose of 15 mg/kg of PD 325901 was chosen as a sub-optimal dose.

Efficacy of the combination of Pegylated Interferon-beta-1a and MEK kinase inhibitor PD325901 in nude mice carrying human SK-MEL-1 melanoma tumors

Athymic nude mice were inoculated with $2X10^6$ SK-MEL-1 human melanoma cells (1 injection in flank; 1 injection per mouse) and 100-200 cubic mm tumors were established. pegylated IFN $\beta$-1a was provided in 20mM acetic acid/sodium acetate at a pH of 4.8 and 150 mM arginine/HCl containing 15 mg/mL human serum albumin (HSA). HSA was used as a control and administered at a concentration of 15 mg/mL. Mice (10 per group) were randomized and treated with either:

  i. PEG400 (once a day (QD) for 14 days) and pegylated Interferon-beta-1a (10 $\mu$g once a week (QW) for 3 weeks);

  ii. PD325901 (15 mg/kg once a day (QD) for 14 days) and pegylated Interferon-beta-1a (10 $\mu$g once a week (QW) for 3 weeks);

  iii. PEG400 (once a day (QD) for 14 days) and (HSA) (QW for 3 weeks);

  iv. PD325901 (15 mg/kg once a day (QD) for 14 days) and HSA (QW for 3 weeks).

Tumors were measured every 3 days using calipers and volume was calculated for a prolate spheroid. The identity of the vehicle control for pegylated interferon-beta-1a vehicle (HSA) and pegylated Interferon-beta-1a was blinded. Tumors were removed 8 hours post last dose and frozen for ERK phosphorylation analysis.

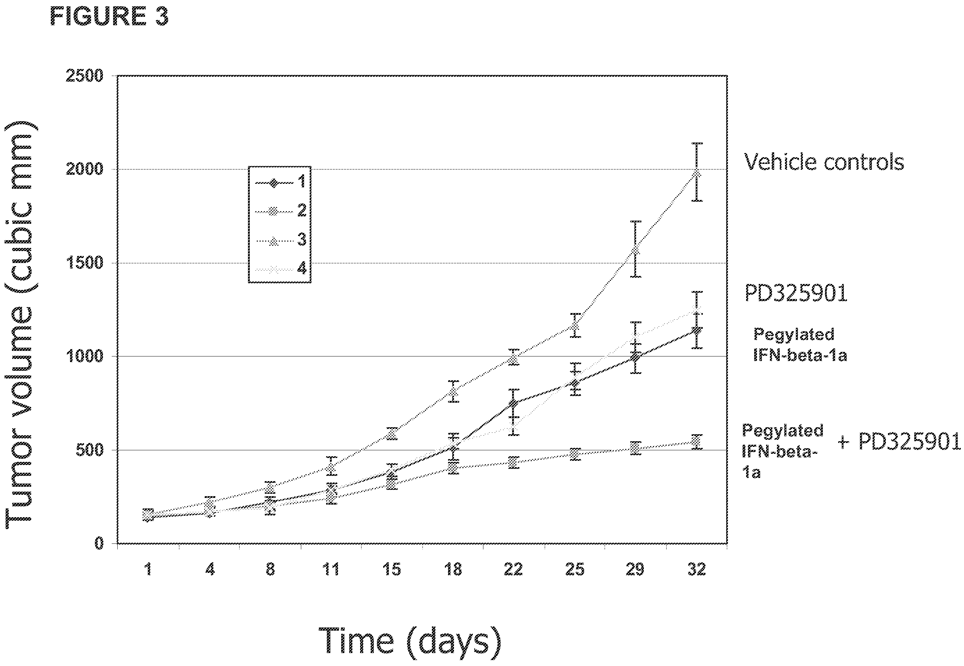

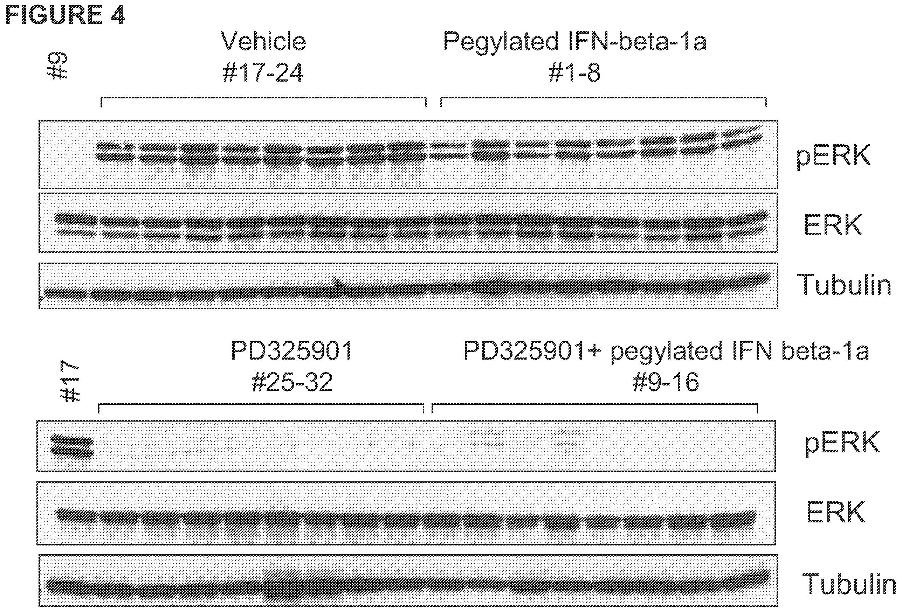

Results: Figure 3 illustrates the effects of vehicle controls, PD325901, pegylated Interferon-beta-1a, pegylated Interferon-beta-1a and PD325901 on SK-MEL-1 tumor volume in nude mice inoculated with SK-MEL-1 human melanoma cells. Figure 3 illustrates a significant difference between all treatment groups and vehicle control group. Figure 3 also shows a significant difference between tumor volume of the mice treated with both pegylated Interferon-beta-1a and PD325901, as compared to either single agent. The difference between estimates of least square means between treatment groups and vehicle control group suggests an additive effect of pegylated Interferon-beta-1a and PD325901: PD325901 (-4.52), pegylated Interferon-beta-1a (-4.77) and pegylated Interferon-beta-1a and PD325901 (-8.63). PD325901, and pegylated Interferon-beta-1a and PD325901 treated groups showed significant inhibition of ERK phosphorylation compared to vehicle and pegylated Interferon-beta-1a treated groups. See Figure 4.

Efficacy of pegylated Interferon-beta-1a alone and in combination with Temozolomide in nude mice carrying human A-375 melanoma tumors

8-10 week old nude mice from Charles River Labs were inoculated subcutaneously with $2X10^6$ A375 human melanoma cells with 50% matrigel in the flank region. When the average tumor volumes reached approximately 200 mm$^3$, the tumor-bearing mice were randomized to treatment groups. Pegylated IFN $\beta$-1a was provided in 20mM acetic acid/sodium acetate at a pH of 4.8 and 150 mM arginine/HCl (4.8A buffer). 20mM acetic acid/sodium acetate at a pH of 4.8 and 150 mM arginine/HCl (4.8A buffer) was used as a vehicle control. Temozolomide was provided in 0.2% hydroxyl propyl methyl cellulose (HPMC) buffer solution. HPMC buffer was used as a vehicle control.

Mice (7-9 per group) were randomized and treated as summarized in Table 1.

Table 1: Study design to investigate the efficacy of pegylated Interferon-beta-1a alone and in combination with Temozolomide in nude mice carrying human A-375 melanoma tumors.

| | Compound | Inoculation conditions | Matrigel (1:1) | Dose (mg/kg) | Rx (route, schedule) | Vehicle/ volume | N |
|---|---|---|---|---|---|---|---|
| 1 | Vehicle PEG-IFN beta-1a | 2 × 10E6 cells/0.2 mL | + | 0 | S.C, BIW × 4 weeks | 4.8A buffer/0.1 mL | 10 |
| 2 | Vehicle TMZ | 2 × 10E6 cells/0.2 mL | + | 5 | P.O, QD × 5 days | 0.2% HPMC/0.1 mL, | 10 |
| 3 | PEG-IFN beta-1a | 2 × 10E6 cells/0.2 mL | + | 0.1 | S.C, BIW × 4 weeks | 4.8A buffer/0.1 mL | 10 |
| 4 | PEG-IFN beta-1a | 2 × 10E6 cells/0.2 mL | + | 0.2 | S.C, BIW × 4 weeks | 4.8A buffer/0.1 mL | 10 |
| 5 | PEG-IFN beta-1a | 2 × 10E6 cells/0.2 mL | + | 0.4 | S.C, BIW × 4 weeks | 4.8A buffer/0.1 mL | 10 |
| 6 | PEG-IFN beta-1a | 2 × 10E6 cells/0.2 mL | + | 0.8 | S.C, BIW × 4 weeks | 4.8A buffer/0.1 mL, | 10 |
| 7 | PEG-IFN beta-1a | 2 × 10E6 cells/0.2 mL | + | 1.6 | S.C, BIW × 4 weeks | 4.8A buffer/0.1 mL, | 10 |
| 8 | TMZ | 2 × 10E6 cells/0.2 mL | + | ++ 5 | P.O, QD × 5 days | 0.2% HPMC/0.1 mL, | 10 |
| 9 | TMZ/ PEG-IFN beta-1a | 2 × 10E6 cells/0.2 mL | + | 5/0.4 | P.O, QD × 5 days/ S.C, BIW × 4 weeks | 4.8A buffer/0.2% HPMC | 10 |

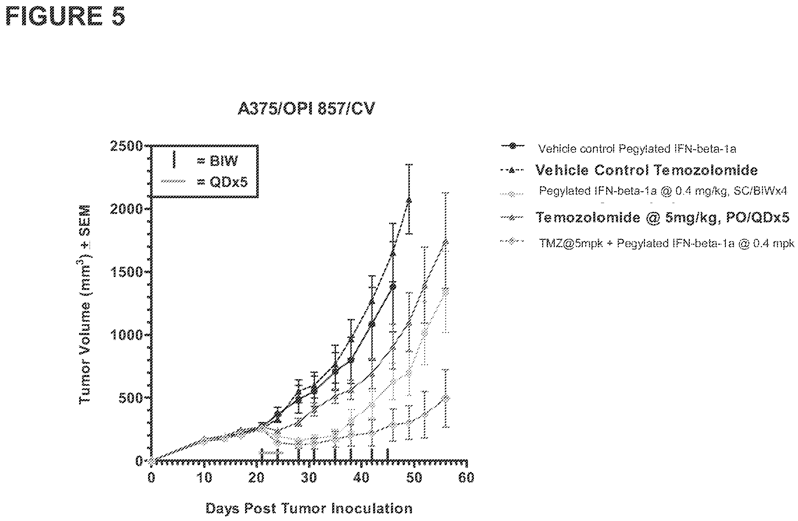

Results: Figure 5 illustrates tumor volume in nude mice of human A-375 melanoma tumors after treatment with vehicle controls, pegylated Interferon-beta-1a alone, temozolomide alone, and pegylated Interferon-beta-1a in combination with temozolomide. As observed, mice treated with the combination of pegylated Interferon-beta-1a in and temozolomide demonstrated a larger inhibition of tumor growth in comparison to mice treated with either agent alone or with vehicle alone.

Tumor growth curves for mice treated with 0.1, 0.2, 0.4, 0.8 or 1.6 mg/kg of pegylated Interferon-beta-1a revealed that tumor growth inhibition increases in mice as the dosages increase (data not shown). However, tumor growth inhibition is almost similar in mice treated with 0.4 or 0.8 mg/kg of pegylated Interferon-beta-1a (data not shown). Additionally, mice treated with 1.6 mg/kg of pegylated Interferon-beta-1a demonstrated an even larger tumor growth inhibition than in mice treated with 0.4 or 0.8 mg/kg of pegylated Interferon-beta-1a (data not shown).

## EXAMPLE 2

Efficacy of pegylated Interferon-beta-1a alone and in combination with Sorafenib, Bevacizumab or Temsirolimus in SCID mice carrying SN12C renal carcinoma tumors.

8-10 week old SCID mice from Charles River Labs were inoculated subcutaneously with $2 \times 10^6$ SN12C renal carcinoma cells with 50 % matrigel in the flank region. When the average tumor volumes reached approximately 250 $mm^3$ the tumor-bearing mice were randomized to treatment groups. Pegylated IFN β-1a was provided in 20mM acetic acid/sodium acetate at a pH of 4.8 and 150 mM arginine/HCl (4.8A buffer). Sorafenib was provided in a Cremophor Ethanol Saline mixture (CES buffer). Bevacizumab was provided in phosphate buffered saline (PBS) buffer. Temsirolimus was provided in buffer containing 0.9% NaCl.

Mice (9-10 per group) were randomized and treated as summarized in Table 2.

Table 2: Study design to investigate the efficacy of pegylated Interferon-beta-1a alone and in combination with Sorafenib, Bevacizumab, and Temsirolimus in SCID mice carrying SN12C renal carcinoma tumors.

| | Compound | Inoculation conditions | Dose (mg/kg) | Rx (route, schedule) | Vehicle/Volume | N |
|---|---|---|---|---|---|---|
| 1 | Vehicle for PEG-IFN beta-1a | 2x10E6 cells in Matrigel | 0 | SC, BIWx7 | Acetate-arginine(4.8A) buffer (0.1 mL) | 10 |
| 2 | Combo Vehicle for Sorafenib and PEG-IFN beta-1a | 2x10E6 cells in Matrigel | 0 | PO, QDx14/SC, BIWx7 | Acetate-arginine(4.8A) buffer (0.1 mL)/CES (0.1 mL) | 10 |
| 3 | Bevacizumab | 2x10E6 cells in Matrigel | 4 | IP, BIWx7 | PBS (0.2 ml) | 10 |
| 4 | Sorafenib | 2x10E6 cells in Matrigel | 60 | PO, QDx14 | CES (0.2 mL) | 10 |
| 5 | Temsirolimus | 2x10E6 cells in Matrigel | 50 | IP, Q7Dx2 | 0.9 % NaCl (0.2 ml) | 10 |
| 6 | Temsirolimus | 2x10E6 cells in Matrigel | 100 | IP, Q7Dx2 | 0.9%NaCl (0.2ml) | 10 |
| 7 | PEG-IFN beta-1a | 2x10E6 cells in Matrigel | 0.8 | SC, BIWx7 | Acetate-arginine(4.8A) buffer (0.1 mL) | 10 |
| 8 | PEG-IFN beta-1a | 2x10E6 cells in Matrigel | 1.6 | SC, BIWx7 | Acetate-arginine(4.8A) buffer (0.1 mL) | 10 |
| 9 | PEG-IFN beta-1a/Bevacizumab | 2x10E6 cells in Matrigel | 0.8/4 | SC, BIWx7/IP, BIWx7 | Acetate-arginine(4.8A) buffer (0.1 mL)/PBS (0.1 ml) | 10 |
| 10 | PEG-IFN beta-la/Sorafenib | 2x10E6 cells in Matrigel | 0.8/60 | SC, BIWx7/PO, QDx14 | Acetate-arginine(4.8A) buffer (0.1 mL)/CES (0.1 mL) | 10 |
| 11 | PEG-IFN beta-la/Sorafenib | 2x10E6 cells in Matrigel | 0.8/60 | SC, BIWx7/PO, QDx14 First Sorafenib followed by PEG-IFN beta-1a | Acetate-arginine(4.8A) buffer (0.1 mL)/CES (0.1 mL) | 10 |
| 12 | PEG-IFN beta-la/Temsirolimus | 2x10E6 cells in Matrigel | 0.8/50 | SC, BIWx7/IP, Q7Dx2 | Acetate-arginine(4.8A) buffer (0.1 mL)/0.9 % NaCl (0.2 ml) | 10 |

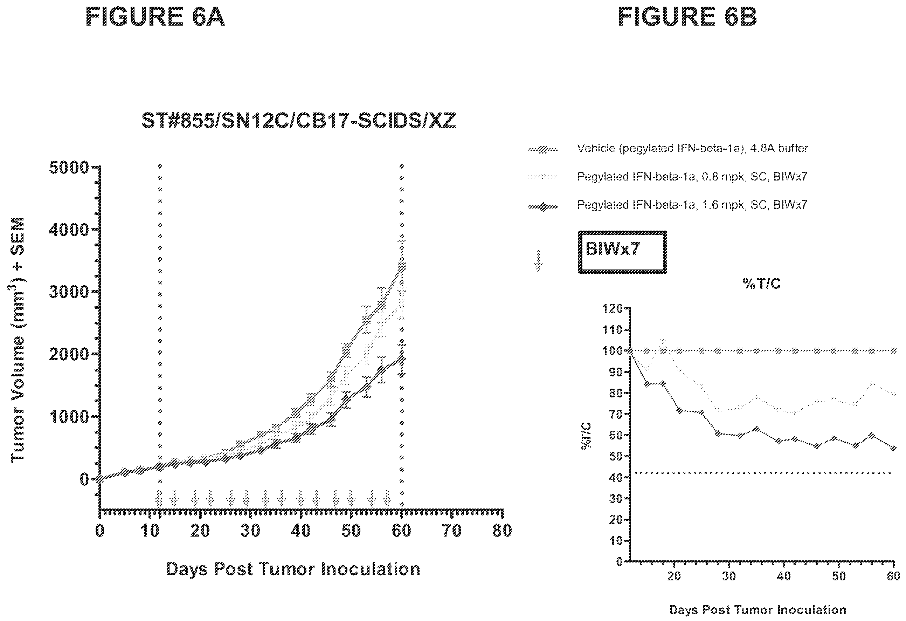

<u>Results:</u> Figure 6A illustrates tumor volume in SCID mice of human SN12C renal carcinoma tumors after treatment with vehicle controls, 0.8 mg/kg or 1.6 mg/kg pegylated Interferon-beta-1a. Figure 6B illustrates %T/C in SCID mice of human SN12C renal carcinoma tumors after treatment with vehicle controls, 0.8 mg/kg or 1.6 mg/kg pegylated Interferon-beta-1a. As observed, the mice treated with pegylated Interferon-beta-1a demonstrated tumor growth inhibition. This is confirmed by repeated measures ANOVA followed by Dunnett's post test (Anova-Dunnett) statistical analysis results showing p-values of $p < 0.05$ for tumor growth inhibition in pegylated interferon beta 1a-treated (at 0.8 mg/kg) versus vehicle-treated mice and $p < 0.001$ for tumor growth inhibition in pegylated interferon beta 1a-treated (at 1.6 mg/kg) versus vehicle-treated mice. Pegylated interferon beta 1a showed statistically significant tumor growth inhibition in a dose dependent manner when dosed at 0.8 mg/kg and 1.6 mg/kg compared to its vehicle.

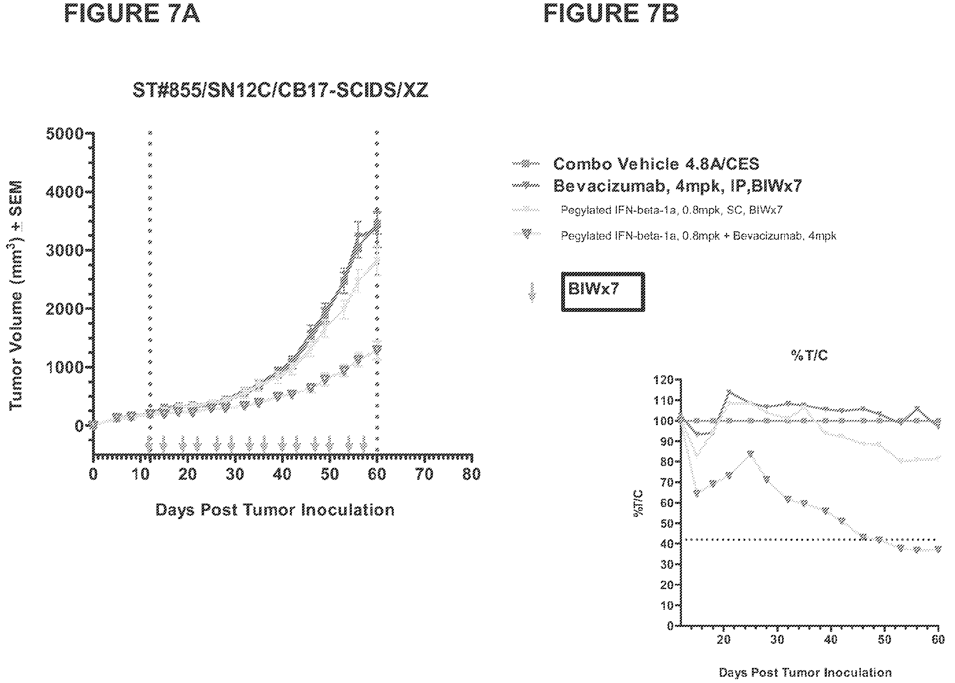

Figure 7A illustrates tumor volume in SCID mice of human SN12C renal carcinoma tumors after treatment with vehicle controls, 4 mg/kg bevacizumab alone, 0.8 mg/kg of pegylated Interferon-beta-1a and a combination of bevacizumab and pegylated Interferon-beta-1a. Figure 7B illustrates %T/C in SCID mice of human SN12C renal carcinoma tumors after treatment with vehicle controls, bevacizumab alone at 4 mg/kg, 0.8 mg/kg of pegylated Interferon-beta-1a and a combination of bevacizumab and pegylated Interferon-beta-1a. As observed, mice treated with a combination of 0.8 mg/kg of pegylated Interferon-beta-1a and 4 mg/kg bevacizumab demonstrated a greater tumor growth inhibition. This is confirmed by repeated measures ANOVA followed by Dunnett's post test (Anova-Dunnett) statistical analysis results showing p-values of $p < 0.01$ for tumor growth inhibition in pegylated interferon beta 1a- and bevacizumab-treated mice versus pegylated interferon beta 1a-treated mice, $p < 0.001$ for tumor growth inhibition in pegylated interferon beta 1a- and bevacizumab-treated mice versus bevacizumab-treated mice, and $p < 0.001$ for tumor growth inhibition in pegylated interferon beta 1a- and bevacizumab-treated mice versus vehicle control-treated mice. Pegylated interferon beta 1a at 0.8 mg/kg dosed in combination with 4 mg/kg bevacizumab showed statistically significant tumor growth inhibition compared to either agent alone.

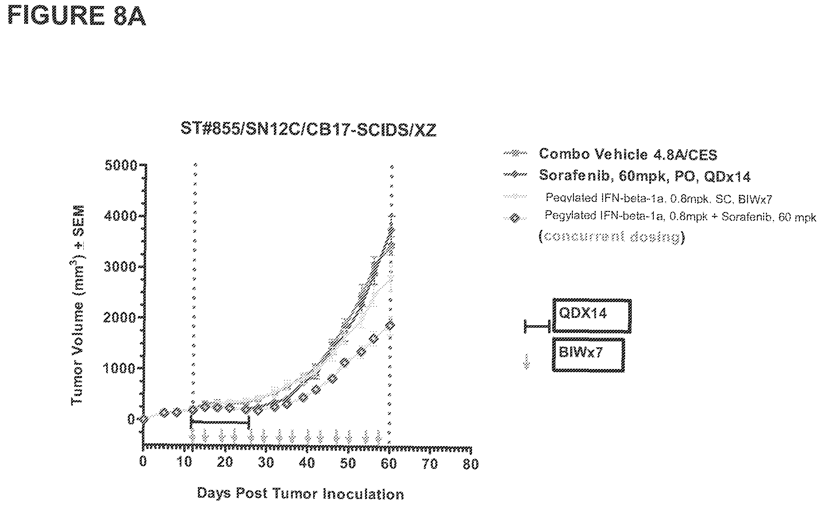

Figure 8A illustrates tumor volume in SCID mice of human SN12C renal carcinoma tumors after treatment with control vehicles, sorafenib at 60 mg/kg, pegylated interferon beta 1a at 0.8 mg/kg and a combination of sorafenib and pegylated interferon beta 1a in a concurrent dosing schedule. As observed, mice treated with a combination of sorafenib and pegylated interferon beta 1a demonstrated a greater tumor growth inhibition. This is confirmed by repeated measures ANOVA followed by Dunnett's post test (Anova-Dunnett) statistical analysis results showing p-values of $p < 0.05$ for tumor growth inhibition in mice treated with a combination of sorafenib and pegylated interferon beta 1a versus pegylated interferon beta 1a alone, $p < 0.01$ for tumor growth inhibition in mice treated with a combination of sorafenib and pegylated interferon beta 1a versus sorafenib alone, $p < 0.001$ for tumor growth inhibition in mice treated with a combination of sorafenib and pegylated interferon beta 1a versus vehicle alone. Concurrent dosing of sorafenib with pegylated interferon beta 1a was significantly superior to treatment with the single agents.

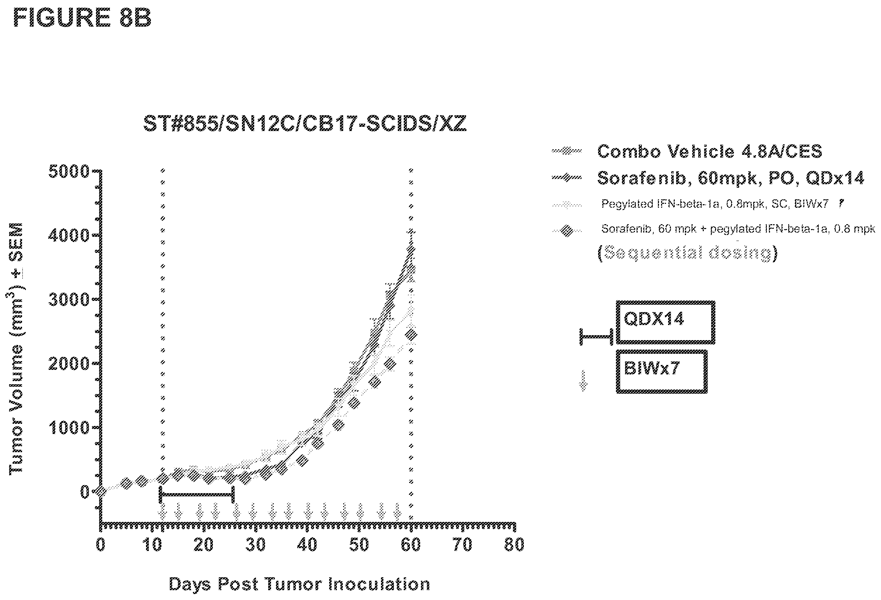

Figure 8B illustrates tumor volume in SCID mice of human SN12C renal carcinoma tumors after treatment with control vehicles, sorafenib alone, pegylated interferon beta 1a alone, and a combination of sorafenib and pegylated interferon beta 1a in a sequential dosing schedule. As observed, mice treated with a combination of sorafenib and pegylated interferon beta 1a demonstrated a greater tumor growth inhibition. This is confirmed by repeated measures ANOVA followed by Dunnett's post test (Anova-Dunnett) statistical analysis results showing p-values of $p < 0.01$ for tumor growth inhibition in mice treated with a combination of sorafenib and pegylated interferon beta 1a versus sorafenib alone, and $p < 0.001$ tumor growth inhibition in mice treated with a combination of sorafenib and pegylated interferon beta 1a versus vehicle alone. Sequential dosing of pegylated interferon beta 1a followed by sorafenib was statistically superior to treatment with sorafenib alone but not different from pegylated interferon beta 1a alone.

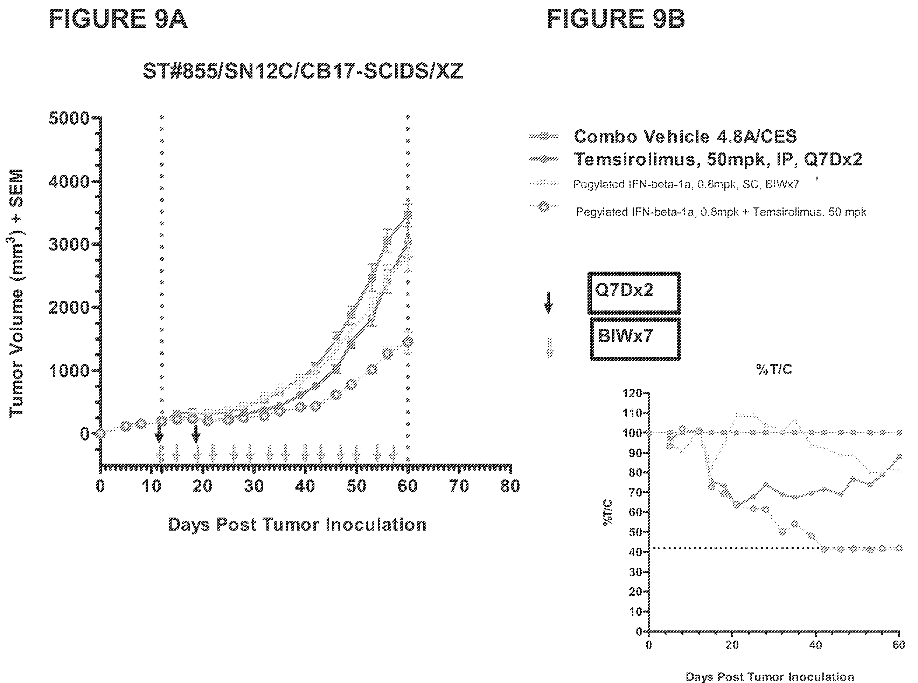

Figure 9A illustrates tumor volume in SCID mice of human SN12C renal carcinoma tumors after treatment with vehicle controls, 50 mg/kg temsirolimus alone, 0.8 mg/kg of pegylated Interferon-beta-1a alone, and a combination of temsirolimus and pegylated Interferon-beta-1a. Figure 9B illustrates %T/C in SCID mice of human SN12C renal carcinoma tumors after treatment with vehicle controls, temsirolimus alone at 50 mg/kg, 0.8 mg/kg of pegylated Interferon-beta-1a and a combination of temsirolimus and pegylated Interferon-beta-1a. As observed, mice treated with a combination of 0.8 mg/kg of pegylated Interferon-beta-1a and 50 mg/kg temsirolimus demonstrated a greater inhibition of tumor growth. This is confirmed by repeated measures ANOVA followed by Dunnett's post test (Anova-Dunnett) statistical analysis results showing p-values of $p < 0.001$ for tumor growth inhibition in pegylated interferon beta 1a- and temsirolimus-treated mice versus vehicle control-treated mice, $p < 0.001$ for tumor growth inhibition in pegylated interferon beta 1a- and temsirolimus-treated mice versus pegylated interferon beta 1a-treated mice, $p < 0.001$ for tumor growth inhibition in pegylated interferon beta 1a- and temsirolimus-treated mice versus temsirolimus-treated mice. Mice treated with pegylated interferon beta 1a (0.8 mg/kg) in combination with temsirolimus showed significantly superior tumor growth inhibition when compared to mice treated with the single agents.

## EXAMPLE 3

Efficacy of pegylated Interferon-beta-1a alone and in combination with Avastin (Bevacizumab) or Irinotecan in nude mice carrying human SW-620 colon carcinoma tumors.

8-10 week old nude mice from Charles River Labs were inoculated subcutaneously with $1 \times 10^6$ SW620 colon carcinoma cells with 50 % matrigel in the flank region. When the average tumor volumes reached approximately 200 mm$^3$, the tumor-bearing mice were randomized to treatment groups. Pegylated IFN $\beta$-1a was provided in 20mM acetic acid/sodium acetate at a pH of 4.8 and 150 mM arginine/HCl (4.8A buffer). Mice were randomized and treated as summarized in Table 3.

Table 3: Study design to investigate the efficacy of pegylated Interferon-beta-1a alone and in combination with paclitaxel in nude mice carrying human SW620 colon carcinoma tumors.

| | Compound | Inoculation conditions | Matrigel (1:1) | Dose (mg/kg) | Rx (route, schedule) | Vehicule/volume | N |
|---|---|---|---|---|---|---|---|
| 1 | Vehicle PEG-IFN-beta-1a | $1 \times 10E6$ cells/0.2 mL | + | 0 | S.C, BIW $\times$ 4 weeks | 4.8A buffer/0.1 mL | 10 |
| 2 | Vehicle Irinotecan | | | 0 | I.P., QD $\times$ 10 days | Saline/0.1 mL | 10 |
| 3 | Vehicle Combo | | | 0/0 | S.C, BIW $\times$ 4 weeks/I.P., QD $\times$ 10 days | 4.8A buffer/0.1 mL/ Saline/0.1 mL | 10 |
| 4 | Bevacizumab | | | 4 | I.P.,BIWx4 | PBS/0.2 ml | 10 |
| 5 | PEG-IFN-beta-1a | | | 0.4 | S.C, BIW $\times$ 4 weeks | 4.8A buffer/0.1 mL | 10 |
| 6 | PEG-IFN-beta-1a | | | 0.8 | S.C, BIW $\times$ 4 weeks | 4.8A buffer/0.1 mL | 10 |
| 7 | PEG-IFN-beta-1a | | | 1.6 | S.C, BIW $\times$ 4 weeks | 4.8A buffer/0.1 mL | 10 |
| 8 | Irinotecan | | | 10 | I.P., QD $\times$ 10 days | Saline/0.1 mL | 10 |
| 9 | Irinotecan/PEG-IFN-beta-1a | | | 10/0.8 | I.P, QD $\times$ 10 days/S.C, BIW $\times$ 4 weeks | Saline 0.1mL/4.8A buffer 0.1mL | 10 |
| 10 | Bevacizumab/PEG-IFN-beta-1a | | | 4/0.8 | I.P.,BIWx4/ S.C, BIW $\times$ 4 weeks | PBS 0.1mL/4.8A buffer 0.1mL | 10 |

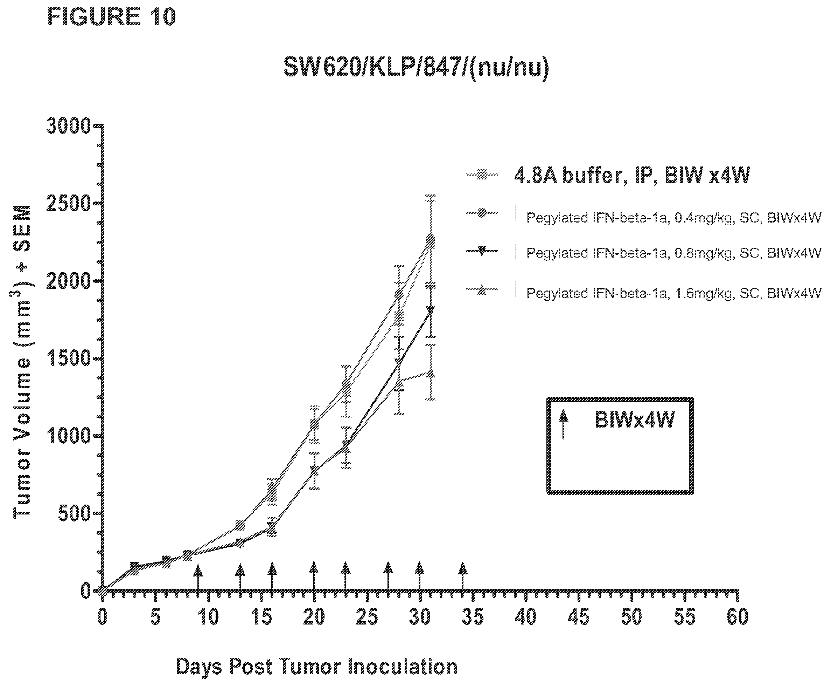

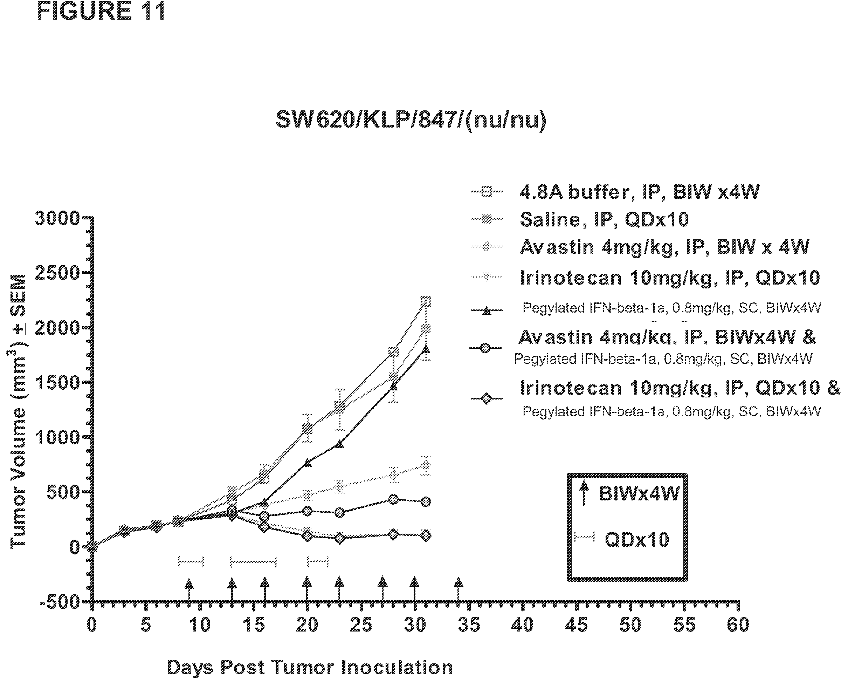

Results: Figure 10 illustrates tumor volume in nude mice of human SW-620 colon carcinoma tumors after treatment with vehicle control (4.8A buffer), and pegylated IFN-beta-1a at concentrations of 0.4 mg/kg, 0.8 mg/kg and 1.6 mg/kg. Mice were injected subcutaneously twice a week (BIW) for 4 weeks. As observed, the inhibition of tumor growth is dose dependent. Figure 11 illustrates tumor volume in nude mice of human SW-620 colon carcinoma tumors after treatment with vehicle controls, avastin (bevacizumab) alone, irinotecan alone, pegylated Interferon-beta-1a alone, a combination of avastin and pegylated Interferon-beta-1a, and a combination of irinotecan and pegylated Interferon-beta-1a. As observed, mice treated with a combination of 0.8 mg/kg of pegylated Interferon-beta-1a and 4 mg/kg Avastin demonstrated a greater inhibition of tumor growth compared to mice treated with either agent alone. In contrast, mice treated with a combination of 0.8 mg/kg of pegylated Interferon-beta-1a and 10mg/kg irinotecan demonstrated the same tumor growth inhibition as mice treated with irinotecan alone.

## EXAMPLE 4

Efficacy of pegylated Interferon-beta-1a alone and in combination with paclitaxel in SCID mice carrying human MDA-MB-231 breast carcinoma tumors.

8-10 week old SCID mice from Charles River Labs were inoculated subcutaneously with $5 \times 10^6$ MDA-MB-231 breast carcinoma cells with 50 % matrigel in the flank region. When the average tumor volumes reached approximately 200 $mm^3$ the tumor-bearing mice were randomized to treatment groups. Pegylated IFN $\beta$-1a was provided in 20mM acetic acid/sodium acetate at a pH of 4.8 and 150 mM arginine/HCl (4,8A buffer). Paclitaxel was provided in a Cremophor Ethanol Saline mixture (CES buffer).

Mice (10 per group) were randomized and treated as summarized in Table 4.

Table 4: Study design to investigate the efficacy of pegylated Interferon-beta-1a alone and in combination with paclitaxel in SCID mice carrying human MDA-MB-231 breast carcinoma tumors.

| | Compound | Inoculation conditions | Matrigel | Dose (mg/kg) | Rx (route, schedule) | Vehicle | N |
|---|---|---|---|---|---|---|---|
| 1 | Vehicle for PEG-IFN beta-1a | $5 \times 10^6/0.2$ ml | 50% | 0 | S.C., BIW | 4.8A buffer/0.1 mL | 10 |
| 2 | Vehicle for Paclitaxel | $5 \times 10^6/0.2$ ml | 50% | 0 | I.P., q4dx3 | Cremophor/Ethanol | 10 |
| 3 | Combo Vehicle for PEG-IFN beta-1a and Paclitaxel | $5 \times 10^6/0.2$ ml | 50% | 0/0 | S.C., BIW/I.P., q4dx3 | 4.8A buffer/0.1 mL/ Cremophor/Ethanol | 10 |
| 4 | Paclitaxel | $5 \times 10^6/0.2$ ml | 50% | 25 | I.P., q4dx3 | Cremophor/Ethanol | 10 |
| 5 | PEG-IFN beta-1a | $5 \times 10^6/0.2$ ml | 50% | 0.4 | SC, BIW | 4.8A buffer/0.1 mL | 10 |
| 6 | PEG-IFN beta-1a | $5 \times 10^6/0.2$ ml | 50% | 0.8 | SC, BIW | 4.8A buffer/0.1 mL | 10 |
| 7 | PEG-IFN beta-1a | $5 \times 10^6/0.2$ ml | 50% | 1.6 | SC, BIW | 4.8A buffer/0.1 mL | 10 |
| 8 | BIIB017/Paclitaxel | $5 \times 10^6/0.2$ ml | 50% | 0.8/25 | SC, BIW/I.P., q4dx3 | 4.8A buffer/0.1 mL/ Cremophor/Ethanol | 10 |
| 9 | BIIB017/Paclitaxel | $5 \times 10^6/0.2$ ml | 50% | 1.6/25 | SC, BIW/I.P., q4dx3 | 4.8A buffer/0.1 mL/ Cremophor/Ethanol | 10 |

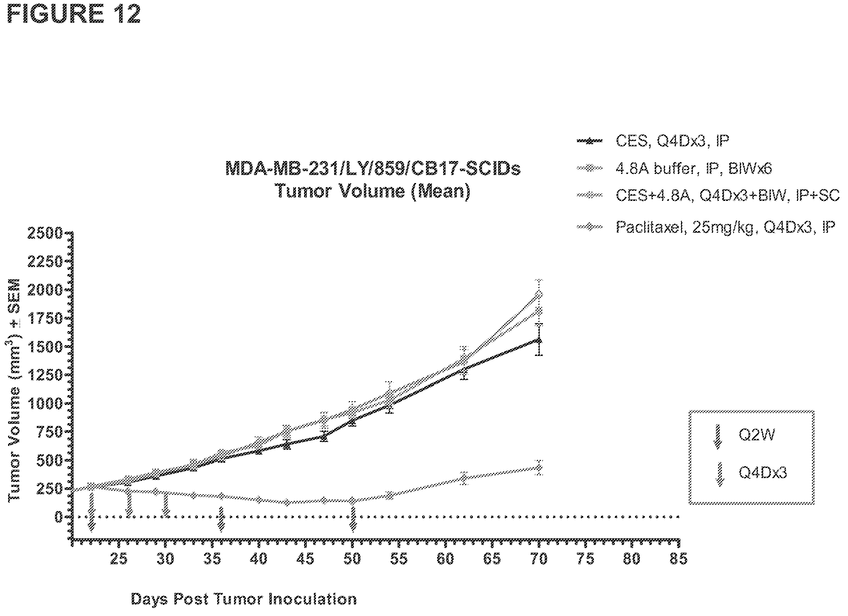

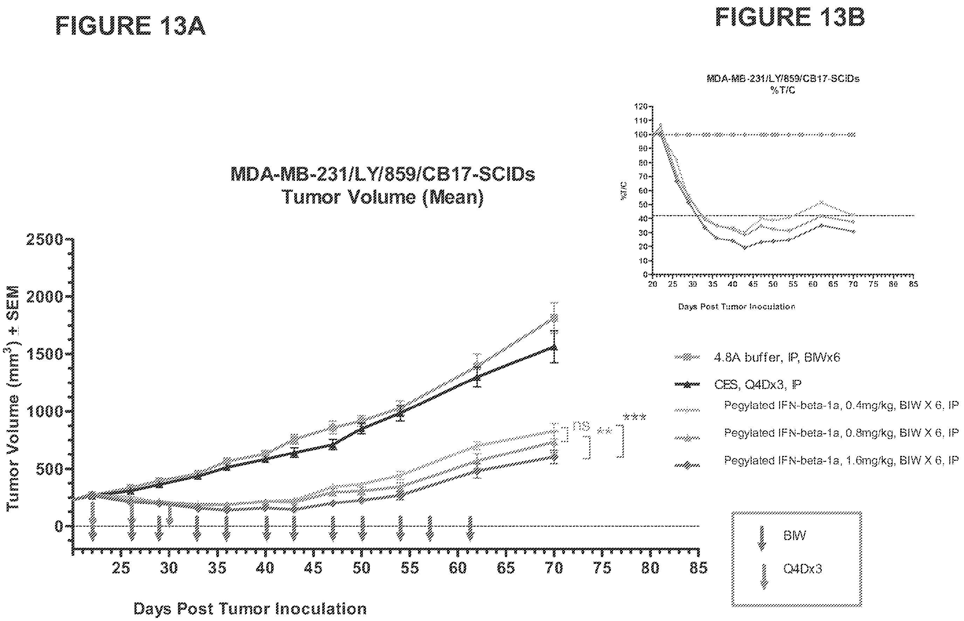

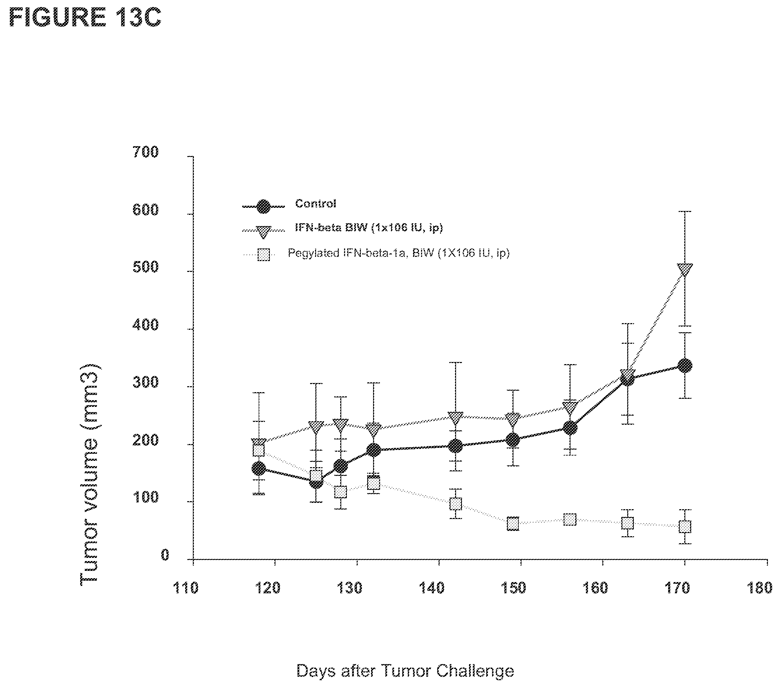

Results: Figure 12 illustrates tumor volume in SCID mice of human MDA-MB-231 breast carcinoma tumors treated with vehicle controls and paclitaxel. As observed, mice treated with paclitaxel demonstrated a greater inhibition of tumor growth compared to mice treated with vehicle controls. Figure 13A illustrates tumor volume in SCID mice of human MDA-MB-231 breast carcinoma tumors treated with vehicle controls and pegylated IFN-beta-1a at 0.4 mg/kg, 0.8 mg/kg and 1.6 mg/kg doses. Figure 13B summarizes %T/C in SCID mice of human MDA-MB-231 breast carcinoma tumors treated with vehicle controls and pegylated IFN-beta-1a at 0.4 mg/kg, 0.8 mg/kg and 1.6 mg/kg doses. As observed, mice treated with pegylated interferon beta 1a at doses ranging from 0.4 to 1.6 mg/kg demonstrated a greater inhibition of tumor growth compared to mice treated with either vehicle controls. Figure 13C illustrates tumor volume in nude mice of human MDA-MB-468 breast carcinoma tumors treated with vehicle control, IFN-beta-1a, or pegylated IFN-beta-1a. As observed, mice treated with pegylated IFN-beta-1a demonstrated a greater inhibition of tumor growth compared to mice treated with IFN-beta-1a or vehicle control.

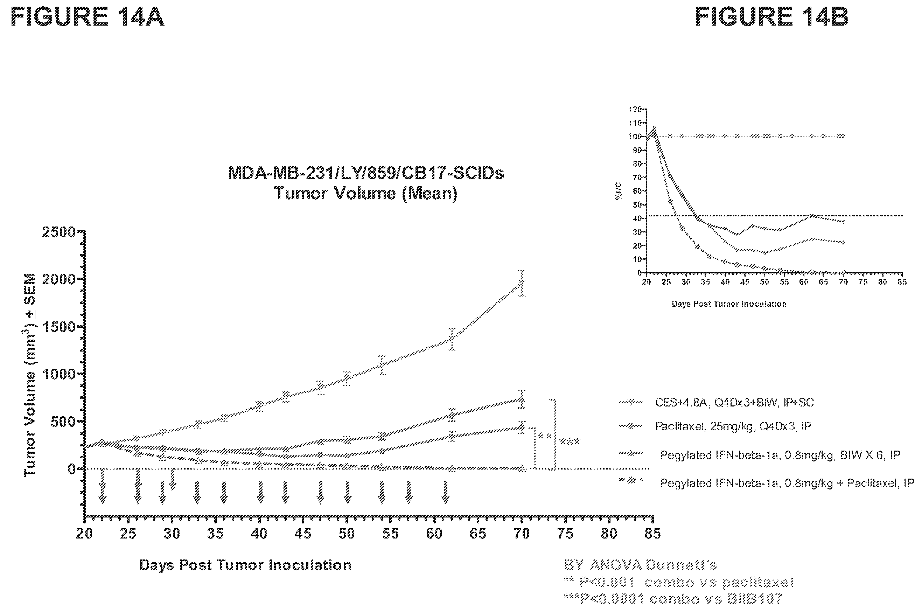

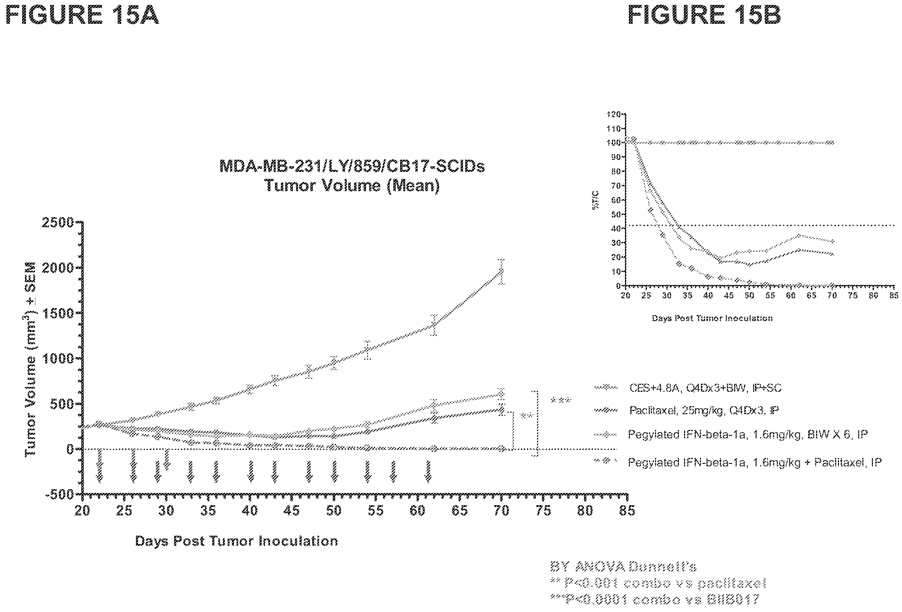

Figure 14A illustrates tumor volume in SCID mice of human MDA-MB-231 breast carcinoma tumors treated with vehicle control, 0.8 mg/kg pegylated IFN-beta-1a alone, 25 mg/kg paclitaxel alone, and a combination of 0.8 mg/kg pegylated IFN-beta-1a and 25 mg/kg paclitaxel. Figure 14B summarizes %T/C in SCID mice of human MDA-MB-231 breast carcinoma tumors treated with vehicle control, 0.8 mg/kg pegylated IFN-beta-1a alone, 25 mg/kg paclitaxel alone and a combination of 0.8 mg/kg pegylated IFN-beta-1a and 25 mg/kg paclitaxel. Figure 15A illustrates tumor volume in SCID mice of human MDA-MB-231 breast carcinoma tumors treated with vehicle control, 1.6 mg/kg pegylated IFN-beta-1a alone, 25 mg/kg paclitaxel alone, and a combination of 1.6 mg/kg pegylated IFN-beta-1a and 25 mg/kg paclitaxel. Figure 15B summarizes %T/C in SCID mice of human MDA-MB-231 breast carcinoma tumors treated with vehicle control, 1.6 mg/kg pegylated IFN-beta-1a alone, 25 mg/kg paclitaxel alone and a combination of 1.6 mg/kg pegylated IFN-beta-1a and 25 mg/kg paclitaxel. As observed in Figures 14 and 15, the combination of pegylated interferon beta 1a at either 0.8 mg/kg or 1.6 mg/kg with paclitaxel demonstrated statistically superior tumor growth inhibition compared treatment with the single agents. This is confirmed by repeated measures ANOVA followed by Dunnett's post test (Anova-Dunnett) statistical analysis results showing p-values of $p < 0.001$ for tumor growth inhibition in pegylated interferon beta 1a- and paclitaxel-treated mice versus paclitaxel-treated mice, and $p < 0.0001$ for tumor growth inhibition in pegylated interferon beta 1a- and paclitaxel-treated mice versus pegylated interferon beta 1a-treated mice.

**EXAMPLE 5**

Efficacy of pegylated IFN-beta-1a in nude mice carrying human WM-266-4 melanoma

8-10 week old nude mice from Charles River Labs were inoculated subcutaneously with 2 x $10^6$ WM-266-4 melanoma cells with 50 % matrigel in the flank region. When the average tumor volumes reached approximately 400 mm$^3$ the tumor-bearing mice were randomized to treatment groups. Nude mice with WM-266-4 tumors (400 cubic mm) were dosed with vehicle control, 0.1, 0.2, 0.4, or 0.8 mg/kg PEG IFN beta-1a SC BIW for 4 weeks with post dose follow up. The mice were examined for dose-dependent tumor inhibition/regression and tumor regrowth during follow up. In another experiment, nude mice with WM-266-4 tumors (200 mm3) were dosed with vehicle control, 0.4, 0.8, or 1.6 mg/kg PEG IFN beta-1a SC once a week (QW), twice a week (BIW), or three times a week (TIW) for 4 weeks with post-dose follow up. In the same study vehicle-treated control tumors were allowed to grow to an average of 2000 mm$^3$. Mice were then treated with 0.8, or 1.6 mg/kg PEG IFN beta-1a on day 46 twice a week for 4 weeks (BIWX4).

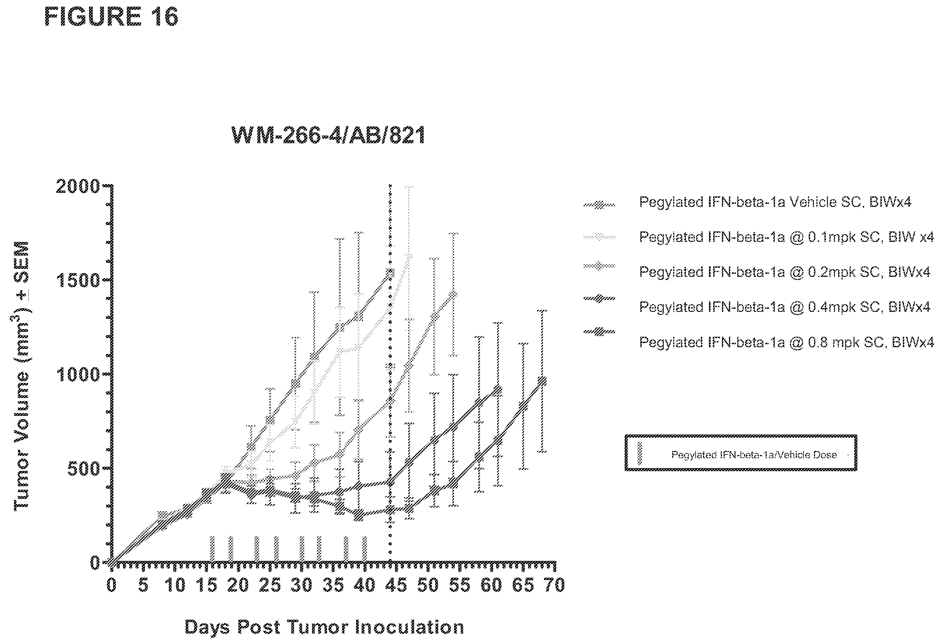

Results: Figure 16 illustrates tumor volume in nude mice of human WM-266-4 melanoma tumors treated with vehicle control, and pegylated interferon-beta-1a at 0.1 mg/kg, 0.2 mg/kg, 0.4 mg/kg and 0.8 mg/kg. As observed, there is significant difference ($p<0.001$) in tumor inbihition between vehicle control-treated mice and mice treated with 0.2-0.8 mg/kg doses. The mice also exhibited a dose-dependent tumor inhibition/regression on drug and upon tumor regrowth.

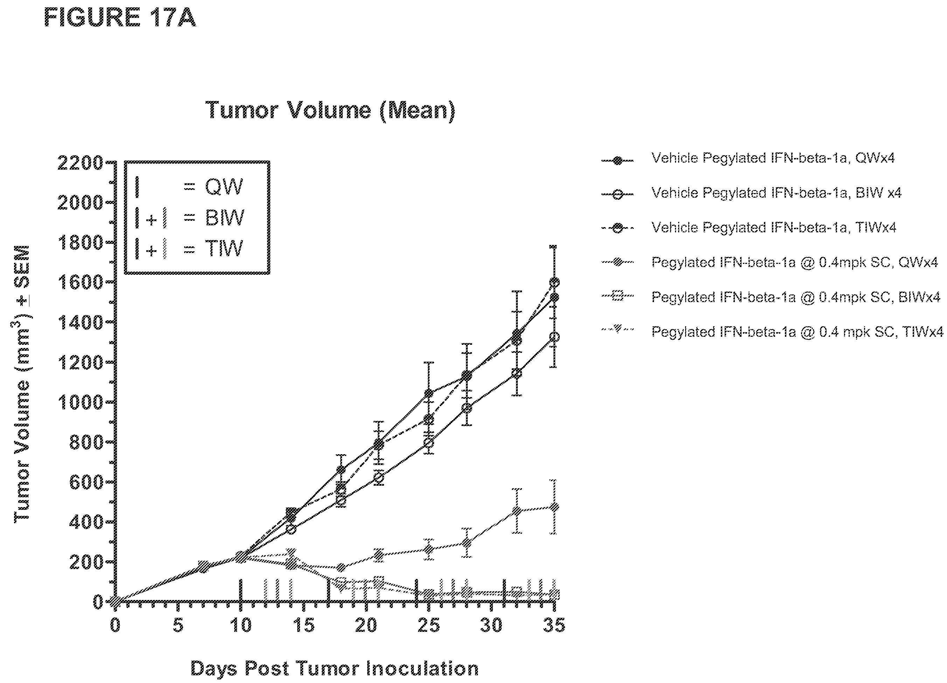

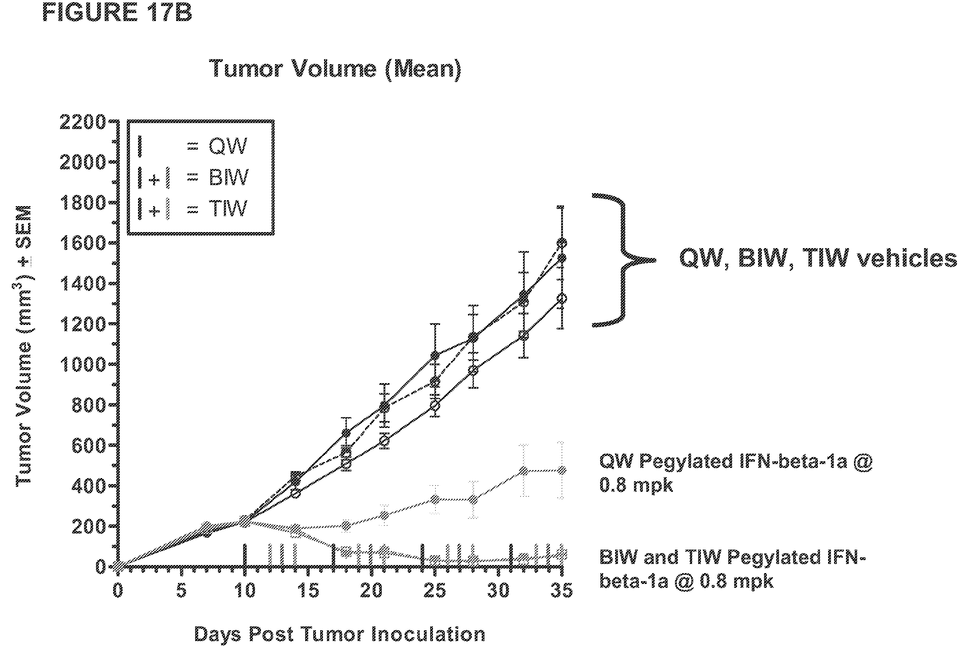

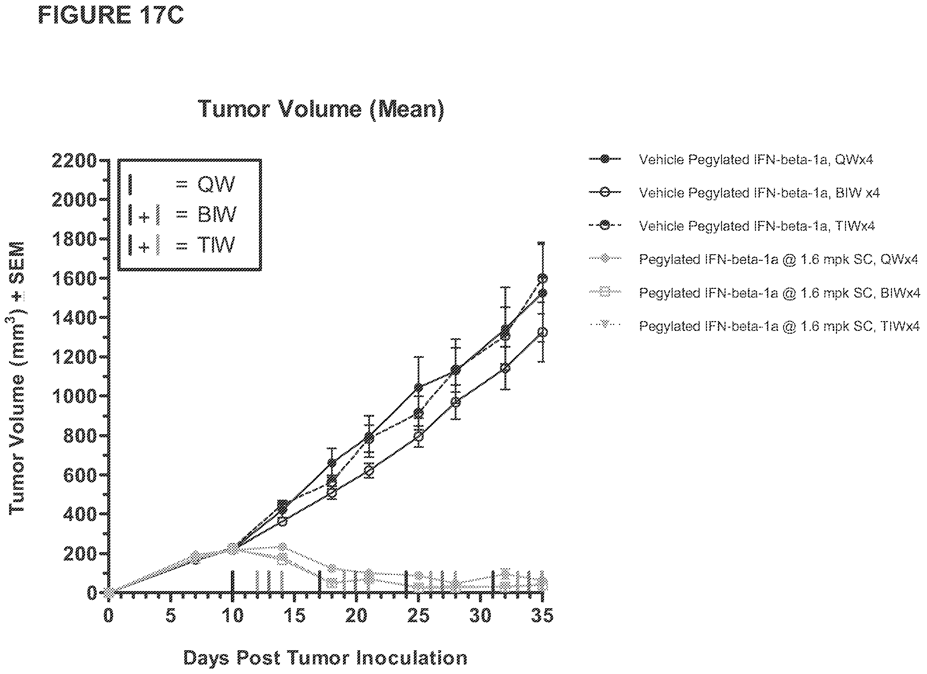

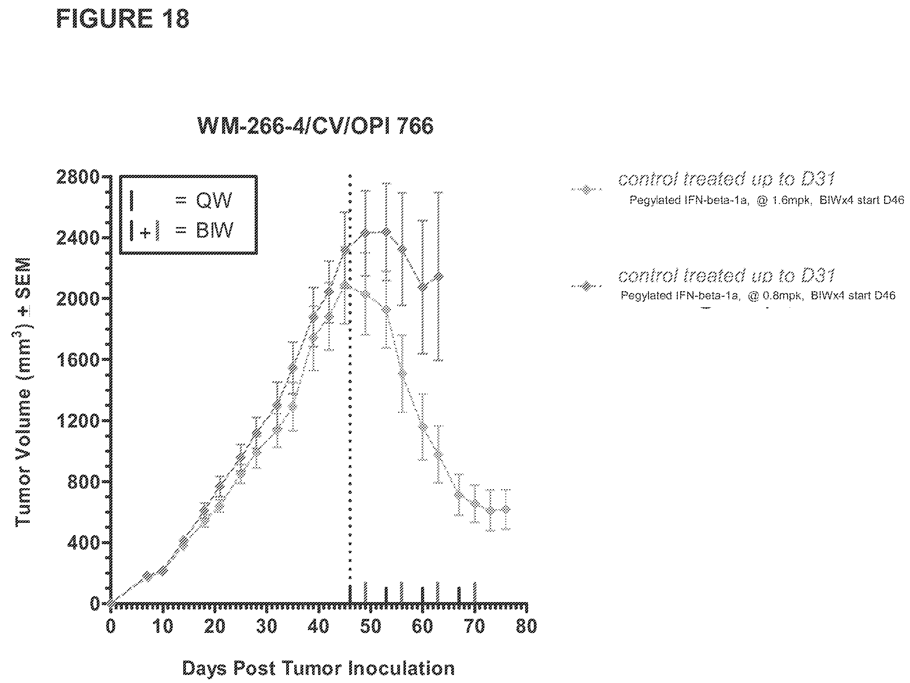

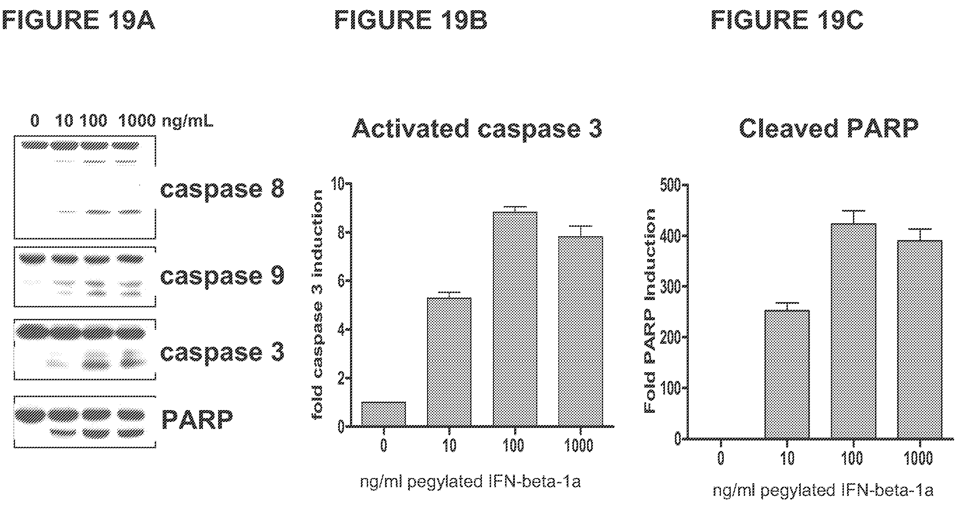

Figures 17A-C illustrates tumor volume in nude mice of human WM-266-4 melanoma tumors treated with vehicle control, 0.4mg/kg, 0.8 mg/kg or 1.6 mg/kg pegylated interferon-beta-1a once a week (QW), twice a week (BIW), or three times a week (TIW). The mice exhibited a dose-dependent tumor inhibition/regression on drug and upon tumor regrowth. Tumor regression of large tumors (2000-2400 cubic mm) was also observed in these experiments. See Figure 18. Figures 19 A-C illustrates the effect on caspase activation and PARP cleavage in WM-266-4 melanoma cells treated with pegylated interferon-beta-1a for 48 hours.

**Claims**

1. Pegylated interferon-beta-1a for use in a method of treating breast carcinoma, wherein the treatment comprises administering said pegylated interferon-beta-la to a subject in combination with a mitotic inhibitor.

2. A mitotic inhibitor for use in a method of treating breast carcinoma, wherein the treatment comprises administering said mitotic inhibitor to a subject in combination with pegylated interferon-beta-1a.

3. Pegylated interferon-beta-la and a mitotic inhibitor for use in a method of treating breast carcinoma, wherein the treatment comprises administering said pegylated interferon-beta-la and mitotic inhibitor to a subject in combination.

4. Pegylated interferon-beta-la for use according to claim 1, a mitotic inhibitor for use according to claim 2, or pegylated interferon-beta-la and a mitotic inhibitor for use according to claim 3, wherein the mitotic inhibitor is paclitaxel.

5. Pegylated interferon-beta-la for use according to claim 1 or claim 4, a mitotic inhibitor for use according to claim 2 or claim 4, or pegylated interferon-beta-la and a mitotic inhibitor for use according to claim 3 or claim 4, wherein the pegylated interferon-beta-la is an interferon-beta-la modified at the N-terminal $\alpha$-amino group with a single, linear 20 kDa mPEG-O-2-methylpropionaldehyde group.

**Patentansprüche**

1. Pegyliertes Interferon-beta-1a zur Verwendung in einem Verfahren zur Behandlung von Brustkarzinom, wobei die Behandlung Verabreichen des pegylierten Interferon-beta-1a an ein Subjekt in Kombination mit einem Mitoseinhibitor umfasst.

2. Mitoseinhibitor zur Verwendung in einem Verfahren zur Behandlung von Brustkarzinom, wobei die Behandlung Verabreichen des Mitoseinhibitors an ein Subjekt in Kombination mit pegyliertem Interferon-beta-1a umfasst.

3. Pegyliertes Interferon-beta-1a und ein Mitoseinhibitor zur Verwendung in einem Verfahren zur Behandlung von Brustkarzinom, wobei die Behandlung Verabreichen des pegylierten Interferon-beta-1a und des Mitoseinhibitors in Kombination an ein Subjekt umfasst.

4. Pegyliertes Interferon-beta-1a zur Verwendung nach Anspruch 1, ein Mitoseinhibitor zur Verwendung nach Anspruch 2 oder pegyliertes Interferon-beta-1a und ein Mitoseinhibitor zur Verwendung nach Anspruch 3, wobei der Mitoseinhibitor Paclitaxel ist.

5. Pegyliertes Interferon-beta-1a zur Verwendung nach Anspruch 1 oder Anspruch 4, ein Mitoseinhibitor zur Verwendung nach Anspruch 2 oder Anspruch 4 oder pegyliertes Interferon-beta-1a und ein Mitoseinhibitor zur Verwendung nach Anspruch 3 oder Anspruch 4, wobei das pegylierte Interferon-beta-1a ein an der N-terminalen $\alpha$-Aminogruppe mit einer einzelnen, linearen 20 kDa mPEG-O-2-Methylpropionaldehyd-Gruppe modifiziertes Interferon-beta-1a ist.

**Revendications**

1. Interféron bêta-1a pégylé pour une utilisation dans un procédé de traitement du carcinome du sein, dans lequel le traitement comprend l'administration dudit interféron bêta-1a pégylé à un sujet en combinaison avec un inhibiteur de la mitose.

2. Inhibiteur de la mitose pour une utilisation dans un procédé de traitement du carcinome du sein, dans lequel le traitement comprend l'administration dudit inhibiteur de la mitose à un sujet en combinaison avec de l'interféron bêta-1a pégylé.

3. Interféron bêta-1a pégylé et inhibiteur de la mitose pour une utilisation dans un procédé de traitement du carcinome du sein, dans lequel le traitement comprend l'administration dudit interféron bêta-1a pégylé et de l'inhibiteur de la mitose à un sujet en combinaison.

4. Interféron bêta-1a pégylé pour une utilisation selon la revendication 1, inhibiteur de la mitose pour une utilisation selon la revendication 2, ou interféron bêta-1a pégylé et inhibiteur de la mitose pour une utilisation selon la revendication 3, dans lequel l'inhibiteur de la mitose est le paclitaxel.

5. Interféron bêta-1a pégylé pour une utilisation selon la revendication 1 ou la revendication 4, inhibiteur de la mitose pour une utilisation selon la revendication 2 ou la revendication 4, ou interféron bêta-1a pégylé et inhibiteur de la mitose pour une utilisation selon la revendication 3 ou la revendication 4, dans lequel l'interféron bêta-1a pégylé est un interféron bêta-1a modifié au niveau du groupe $\alpha$-amino N-terminal avec un groupe unique linéaire de mPEG-O-2-méthylpropionaldéhyde de 20 kDa.

## FIGURE 1

EP 2 621 516 B1

FIGURE 2

EP 2 621 516 B1

FIGURE 3

EP 2 621 516 B1

# FIGURE 4

Vehicle #17-24 · Pegylated IFN-beta-1a #1-8 · #9 · pERK · ERK · Tubulin

PD325901 #25-32 · PD325901+ pegylated IFN beta-1a #9-16 · #17 · pERK · ERK · Tubulin

EP 2 621 516 B1

# FIGURE 5

A375/OPI 857/CV

## FIGURE 6A

ST#855/SN12C/CB17-SCIDS/XZ

## FIGURE 6B

## FIGURE 7A

ST#855/SN12C/CB17-SCIDS/XZ

Tumor Volume (mm³) ± SEM

Days Post Tumor Inoculation

## FIGURE 7B

- Combo Vehicle 4.8A/CES
- Bevacizumab, 4mpk, IP, BIWx7
- Pegylated IFN-beta-1a, 0.8mpk, SC, BIWx7
- Pegylated IFN-beta-1a, 0.8mpk + Bevacizumab, 4mpk

BIWx7

%T/C

Days Post Tumor Inoculation

# FIGURE 8A

## ST#855/SN12C/CB17-SCIDS/XZ

Legend:
- Combo Vehicle 4.8A/CES
- Sorafenib, 60mpk, PO, QDx14
- Pegylated IFN-beta-1a, 0.8mpk, SC, BIWx7
- Pegylated IFN-beta-1a, 0.8mpk + Sorafenib, 60 mpk
- (concurrent dosing)

QDX14

BIWx7

X-axis: Days Post Tumor Inoculation

Y-axis: Tumor Volume (mm³) ± SEM

EP 2 621 516 B1

# FIGURE 8B

ST#855/SN12C/CB17-SCIDS/XZ

EP 2 621 516 B1

# FIGURE 9A

# FIGURE 9B

## ST#855/SN12C/CB17-SCIDS/XZ

Combo Vehicle 4.8A/CES

Temsirolimus, 50mpk, IP, Q7Dx2

Pegylated IFN-beta-1a, 0.8mpk, SC, BIWx7

Pegylated IFN-beta-1a, 0.8mpk + Temsirolimus, 50 mpk

Q7Dx2

BIWx7

%T/C

EP 2 621 516 B1

FIGURE 10

SW620/KLP/847/(nu/nu)

EP 2 621 516 B1

# FIGURE 11

SW620/KLP/847/(nu/nu)

# FIGURE 12

MDA-MB-231/LY/859/CB17-SCIDs
Tumor Volume (Mean)

Legend:
- CES, Q4Dx3, IP
- 4.8A buffer, IP, BIWx6
- CES+4.8A, Q4Dx3+BIW, IP+SC
- Paclitaxel, 25mg/kg, Q4Dx3, IP

Q2W

Q4Dx3

X-axis: Days Post Tumor Inoculation
Y-axis: Tumor Volume (mm$^3$) ± SEM

EP 2 621 516 B1

# FIGURE 13A

# FIGURE 13B

**MDA-MB-231/LY/859/CB17-SCIDs**
**Tumor Volume (Mean)**

MDA-MB-231/LY/859/CB17-SCIDs
%T/C

4.8A buffer, IP, BIWx6

CES, Q4Dx3, IP

Pegylated IFN-beta-1a, 0.4mg/kg, BIW X 6, IP

Pegylated IFN-beta-1a, 0.8mg/kg, BIW X 6, IP

Pegylated IFN-beta-1a, 1.6mg/kg, BIW X 6, IP

BIW

Q4Dx3

# FIGURE 13C

EP 2 621 516 B1

# FIGURE 14A

MDA-MB-231/LУ/859/CB17-SCIDs
Tumor Volume (Mean)

# FIGURE 14B

CES+4.8A, Q4Dx3+BIW, IP+SC
Paclitaxel, 25mg/kg, Q4Dx3, IP
Pegylated IFN-beta-1a, 0.8mg/kg, BIW X 6, IP
Pegylated IFN-beta-1a, 0.8mg/kg + Paclitaxel, IP

BY ANOVA Dunnett's
** $p<0.001$ combo vs paclitaxel
*** $p<0.0001$ combo vs BIIB107

FIGURE 15A

FIGURE 15B

MDA-MB-231/LY/859/CB17-SCIDs
Tumor Volume (Mean)

Days Post Tumor Inoculation

CES+4.8A, Q4Dx3+BIW, IP+SC

Paclitaxel, 25mg/kg, Q4Dx3, IP

Pegylated IFN-beta-1a, 1.6mg/kg, BIW X 6, IP

Pegylated IFN-beta-1a, 1.6mg/kg + Paclitaxel, IP

BY ANOVA Dunnett's
** P<0.001 combo vs paclitaxel
***P<0.0001 combo vs BIIB017

EP 2 621 516 B1

# FIGURE 16

## WM-266-4/AB/821

Legend:
- Pegylated IFN-beta-1a Vehicle SC, BIWx4
- Pegylated IFN-beta-1a @ 0.1mpk SC, BIW x4
- Pegylated IFN-beta-1a @ 0.2mpk SC, BIWx4
- Pegylated IFN-beta-1a @ 0.4mpk SC, BIWx4
- Pegylated IFN-beta-1a @ 0.8 mpk SC, BIWx4

Pegylated IFN-beta-1a/Vehicle Dose

Y-axis: Tumor Volume ($mm^3$) ± SEM

X-axis: Days Post Tumor Inoculation

FIGURE 17A

Tumor Volume (Mean)

Vehicle Pegylated IFN-beta-1a, QWx4
Vehicle Pegylated IFN-beta-1a, BIW x4
Vehicle Pegylated IFN-beta-1a, TIWx4
Pegylated IFN-beta-1a @ 0.4mpk SC, QWx4
Pegylated IFN-beta-1a @ 0.4mpk SC, BIWx4
Pegylated IFN-beta-1a @ 0.4 mpk SC, TIWx4

| = QW
+ | = BIW
+ | = TIW

Tumor Volume (mm$^3$) ± SEM

Days Post Tumor Inoculation

FIGURE 17B

Tumor Volume (Mean)

QW, BIW, TIW vehicles

QW Pegylated IFN-beta-1a @ 0.8 mpk

BIW and TIW Pegylated IFN-beta-1a @ 0.8 mpk

Legend:
| = QW
| + | = BIW
| + | = TIW

Y-axis: Tumor Volume (mm³) + SEM
X-axis: Days Post Tumor Inoculation

## FIGURE 17C

Tumor Volume (Mean)

Y-axis: Tumor Volume (mm³) + SEM

X-axis: Days Post Tumor Inoculation

Legend box:
| = QW
| + | = BIW
| + | = TIW

Legend:
- Vehicle Pegylated IFN-beta-1a, QWx4
- Vehicle Pegylated IFN-beta-1a, BIW x4
- Vehicle Pegylated IFN-beta-1a, TIWx4
- Pegylated IFN-beta-1a @ 1.6 mpk SC, QWx4
- Pegylated IFN-beta-1a @ 1.6 mpk SC, BIWx4
- Pegylated IFN-beta-1a @ 1.6 mpk SC, TIWx4

EP 2 621 516 B1

FIGURE 18

FIGURE 19A

FIGURE 19B

FIGURE 19C

0  10 100  1000 ng/mL

caspase 8

caspase 9

caspase 3

PARP

**Activated caspase 3**

fold caspase 3 induction

ng/ml pegylated IFN-beta-1a

**Cleaved PARP**

Fold PARP Induction

ng/ml pegylated IFN-beta-1a

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4399216 A
- US 5149636 A
- US 5179017 A, Axel
- US 4470461 A, Kaufman
- US 7446173 B
- US 20050107277 A
- US 3710795 A
- US 5399163 A
- US 5383851 A
- US 5312335 A
- US 5064413 A
- US 4941880 A

- US 4790824 A
- US 4596556 A
- US 4487603 A
- US 4486194 A
- US 4447233 A
- US 4447224 A
- US 4439196 A
- US 4475196 A
- US 3773919 A
- US 4767628 A
- WO 9415587 A

**Non-patent literature cited in the description**

- **BAKER DP et al.** *Bioconjugate Chem.,* 2006, vol. 17, 179-188